# EUROPEAN PATENT SPECIFICATION

(11) **EP 3 424 977 B1**
(45) Date of publication and mention of the grant of the patent: **18.03.2020**
(21) Application number: 18179542.8
(22) Date of filing: 25.06.2018
(51) Int. Cl.: C08G 63/685, C08K 5/00, C08G 73/02, C08L 23/12, C08L 77/06, C07D 401/14

(54) **HINDERED POLYMERIC AMINES**
GEHINDERTE POLYMERE AMINE
AMINES POLYMÈRES ENTRAVÉES

(30) Priority: 30.06.2017 IT 201700073726
(43) Date of publication of application: 09.01.2019
(73) Proprietor: 3V SIGMA S.p.A, 20121 Milano (IT)
(72) Inventor: BERTE', Ferruccio, 24121 BERGAMO (IT); MAESTRI, Francesco, 24121 BERGAMO (IT); DEL SORDO, Simone, 24121 BERGAMO (IT)
(74) Representative: Bianchetti Bracco Minoja S.r.l.

(56) References cited:
- WO-A1-2012/055965
- GB-A- 2 202 853
- US-A- 3 901 853

## Description

The present invention relates to polyalkylpiperidine polymer compounds which impart high stability to various kinds of polymer materials, especially polyolefins, towards oxidative action and photodegradation.

### Prior art

Polymers are known to be subject to deterioration due to the action of heat, light and oxygen; these factors cause loss of their mechanical properties, discolouring and other adverse effects.

In order to stabilise polymer materials, mainly towards the UV radiation in sunlight, various classes of compounds have been proposed, such as benzophenone and benzotriazole derivatives. The stability which these compounds give to polymers is acceptable, but insufficient to meet current practical needs, especially in the case of fibres, films and raffia based on olefin polymers.

The polyalkylpiperidine derivatives commonly called HALS (hindered amine light stabilisers) are much more effective, and there are numerous patents relating to them.

Examples of HALS are described in US 4,530,950, DE 1,929,928, US 3,640,928, US 4,477,615, US 4,233,412, US 4,331,586, DE 2,636,144, DE 2,456,864, US 4,315,859, US 4,104,248, US 4,086,204, US 4,038,280, US 4,476,302, US 4,981,964 and EP 2,632,914,
Synergistic mixtures of HALS are described in US 4,692,486, US 4,863,981, US 5,021,485, EP 0709426, EP 0728806 and WO 2012/153260.

However, there is still a need for further compounds or mixtures that possess high stabilising efficacy towards polymer materials.

Particularly useful polyalkylpiperidine compounds are those wherein the active units based on hindered amines are present as pendants of polymer chains.

### Description of the invention

The present invention relates to novel polymer compounds wherein the main polymer chain is a polyester and the pendants are polyalkylpiperidino groups.

Polymer compounds wherein the main chain is a polyester, and the polyalkylpiperidine units are part of the chain, are already known and widely used.

Stabilisers of this type, and their use as light stabilisers in polymers, are described, for example, in US 4,233,412.

Trade names for this type of product, the CAS RN of which is 65447-77-0, are UVASORB HA22 (3V Sigma SpA), Tinuvin 622 (Basf), Lowilite 62 (Addivant) and Light Stabilizer 622, UV-622.

However, in these compounds the activity of the polyalkylpiperidine groups can be partly reduced by the fact that the groups active for light stabilisation are blocked in the polymer chain.

It is therefore desirable to manufacture and use polyester stabilising compounds wherein the groups active for radiation stabilisation are bonded to the main chain as pendants, and therefore more mobile and able to perform a better stabilising action.

A first aspect of the invention relates to compounds of general formula (I): 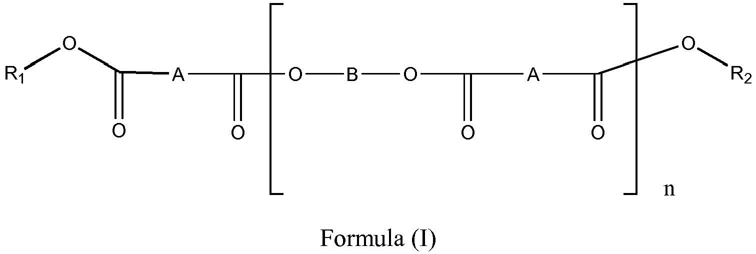 wherein:
n is an integer ranging from 2 to 100;
R₁ and R₂ are hydrogen or saturated, unsaturated, straight or branched C₁-C₁₈ alkyls or aromatic groups.

A is a C₁-C₂₀ alkylene or C₃-C₁₀ cycloalkylene group optionally containing one or more unsaturations or an aromatic group;
wherein "aromatic group" preferably means a phenylene or naphthalene ring, and B is a group of Formula (II): 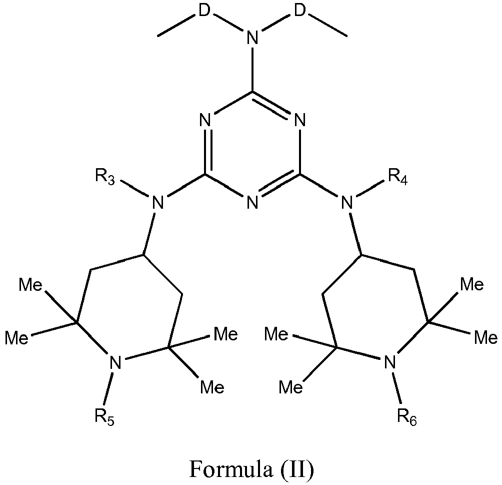 wherein:
D is a C₁-C₆ alkylene or isoalkylene group, optionally unsaturated;
R₃ and R₄, which may be the same or different, are hydrogen or C₁-C₈ alkyl;
R₅ and R₆, which may be the same or different, are hydrogen or straight or branched C₁-C₄ alkyl or an -OG group wherein
G is hydrogen or saturated, unsaturated, straight or branched C₁-C₁₀ alkyl.

The preferred compounds of formula I are those wherein:
n is between 3 and 20,
A is a straight-chain C₁-C₂₀ alkylene group, preferably a -(CH₂)₈- group,
D is a -CH₂-CH₂-, -CH₂-CH₂-CH₂-, -CH(CH₃)-CH₂- or -CH₂-CH(CH₃)- group, preferably a -CH₂-CH₂-, -CH(CH₃)-CH₂- or -CH₂-CH(CH₃)- group
R₁ and R₂ are methyl or ethyl, and are preferably both methyl,
R₃ and R₄ are hydrogen or n-butyl, and are preferably both n-butyl,
R₅ and R₆ are hydrogen or methyl.

The polypiperidine polymer compounds of formula (I) can be obtained by a polycondensation reaction between an intermediate of general formula (III) 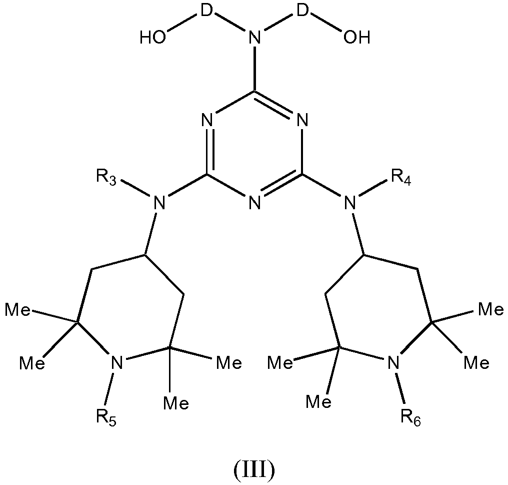 wherein:
D, R₃, R₄, R₅ and R₆ have the meanings above described in formula (II) and compounds of formula (IV) 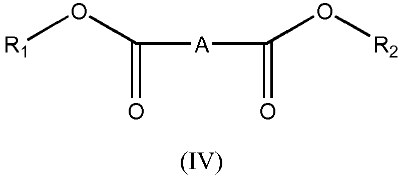 wherein:
R₁, R₂ and A have the meanings above described in formula (I).

The compounds of formula (IV) are used in stoichiometric excess to those of formula III, to ensure that the terminal groups of the polymer chains formed are the ester type described in formula (I). At the end of the polymerisation reaction, the excess of the unreacted compounds of formula (IV) is distilled off under vacuum.

Examples of compounds of formula (IV) are:
- dimethyl esters, diethyl esters, dibutyl esters and dioctyl esters or mixed esters of linear dicarboxylic acids such as propanedioic, butanedioic, hexanedioic, octanedioic, decanedioic and dodecanedioic acids;
- dimethyl esters, diethyl esters, dibutyl esters and dioctyl esters or mixed esters of aromatic dicarboxylic acids such as terephthalic acids and phthalic acid;
- dimethyl esters, diethyl esters, dibutyl esters and dioctyl esters of cycloalkane dicarboxylic acids such as 1,2-dicarboxycyclohexane, 1,3-dicarboxycyclohexane and 1,4-dicarboxycyclohexane.

The intermediates of formula (III) can easily be synthesised by well-known methods by reacting cyanuryl chloride with an equivalent of each of the compounds of formulas (V), (VI) and (VII) as described, for example, in JPS58152881: 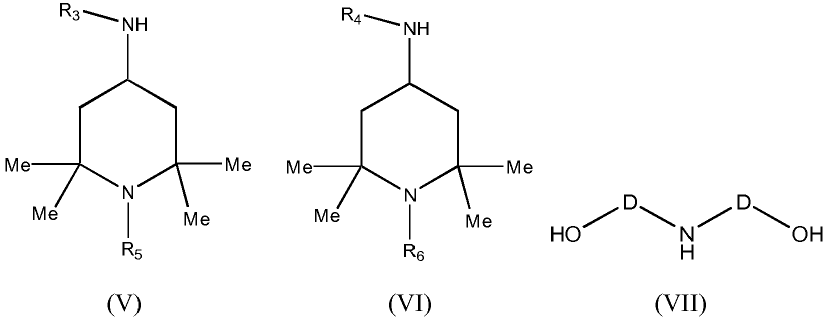 wherein D, R₃, R₄, R₅, R₆ and D have the meanings above described in formula II.

The synthesis conditions of the compounds of formula (I) are those typical of the polycondensation reactions used to prepare polyesters. A typical procedure for the preparation of linear polyesters consists of polycondensation of diols with dicarboxylic acids in the presence of suitable catalysts, with the removal of the water released during the esterification reaction. Similarly, linear polyesters can also be obtained by polycondensation of diols and diesters of dicarboxylic acids in the presence of suitable trans-esterification catalysts. In this case, the polymerisation reaction is conducted by removing the alcohols released during the reaction, which are generally low-boiling. The reaction is usually conducted at temperatures ranging between 50 and 300°C, preferably between 100 and 200°C, and at pressures ranging between 0 and 2 bars, preferably operating under vacuum.

The polyesters can be prepared in bulk or in the presence of suitable solvents inert to the esterification reaction. Examples of suitable solvents are saturated or aromatic hydrocarbons such as heptane, decane, toluene and xylene, ketones such as cyclohexanone and methyl-isobutylketone, and nitriles such as benzonitrile. Typical catalysts can be acids such as sulphuric acid, methanesulphonic acid and p-toluenesulphonic acid, bases such as lithium amide and sodium methylate, alkyl titanates such as tetraisopropyl titanate, and tin compounds such as dibutyltin oxide.

The polymerisation reactions are conducted with a well-determined stoichiometric excess of the compound of formula (IV) and with high reaction conversions, with the aim of obtaining polymer chains of a defined molecular weight terminating with ester groups at both ends.

The compounds of formula (I) are efficient light stabilisers for polymer materials, especially for polyolefins, either used alone or mixed with many other known stabilisers.

Mixtures of the compounds of formula (I) with other known stabilisers are also part of the invention.

In particular, mixtures of the compounds of formula (I) with at least one of the compounds of formula **(P), (Q), (R), (S), (T)** or **(U)** are preferred. 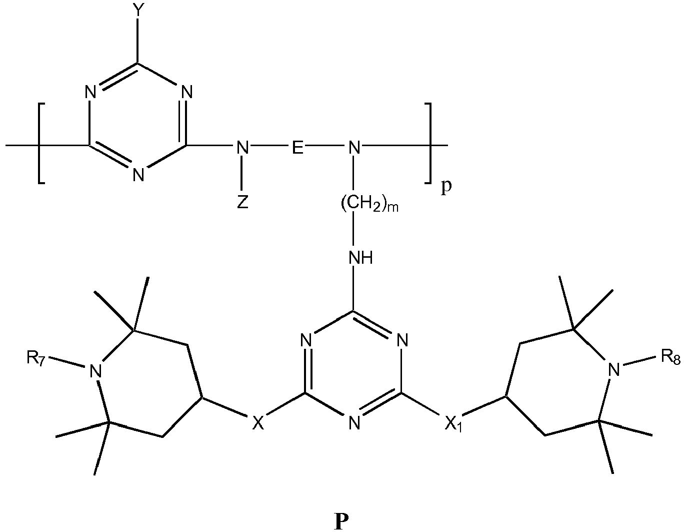
wherein p is between 3 and 20;
m is between 2 and 12;
R₇ and R₈, which may be the same or different, represent hydrogen, a straight or branched C₁-C₁₂ alkyl group, a C₃-C₈ alkenyl group or a C₇-C₁₉ aralkyl group;
X and X₁, which may be the same or different, represent oxygen or a group of formula (VIII) 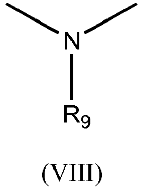 wherein R₉ is hydrogen, a straight or branched C₁-C₁₂ alkyl group, a C₅-C₁₂ cycloalkyl group or a C₇-C₁₂ aralkyl group;
E represents a -(CH₂)ₐ- group wherein a is between 2 and 12, on the proviso that a is different from m;
**Z** represents a C₁-C₁₈ alkyl group or a group of formula (IX) 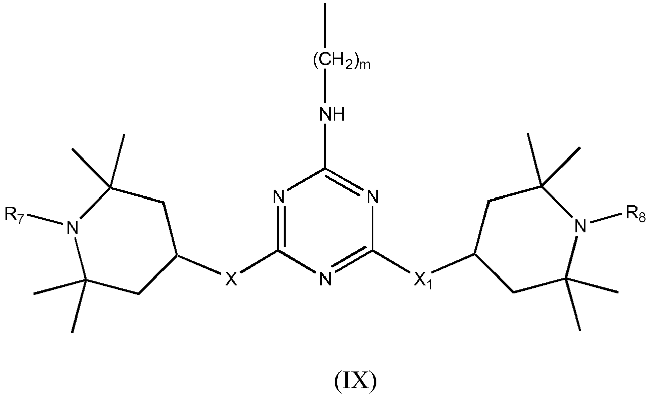 wherein m, X, X₁, R₇ and R₈ are as defined above,
   or a group of formula (X) 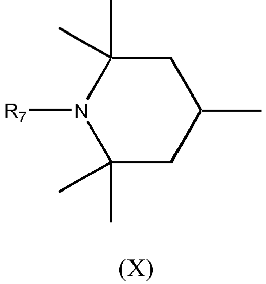 wherein R7 is as defined above;
Y represents the O-R₁₁ and S-R₁₁ groups or a group of formula (XI) 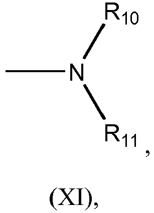 wherein R₁₀ and R₁₁, which may be the same or different, are hydrogen, a straight or branched C₁-C₁₈ alkyl group, a C₅-C₁₂ cycloalkyl group, a C₇-C₁₂ aralkyl group or a C₆-C₁₂ aryl group, or can form, together with the nitrogen atom to which they are bonded, a morpholino group or a C₅-C₇ heterocycle;
   and the piperidino group (XII) 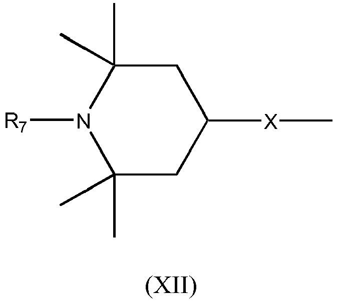 wherein R₇ and X are as defined above; 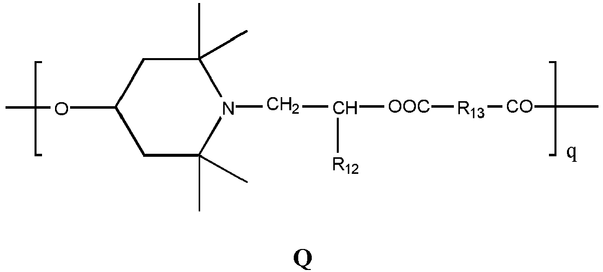 wherein
   R₁₂ is hydrogen or methyl;
   R₁₃ is a direct bond or a C₁-C₁₀ alkylene group;
   q is an integer ranging from 2 to 50; 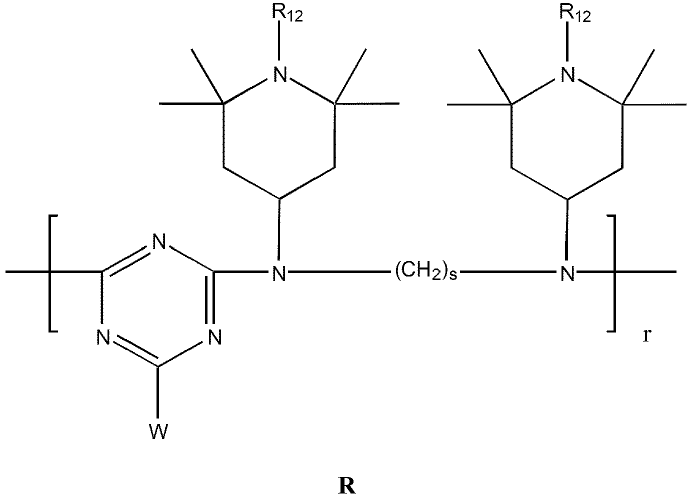
   wherein:
   r is an integer ranging from 2 to 50
   s is an integer ranging from 2 to 10
   R₁₂ is as defined above for the compounds of Formula Q;
W is a group of formula (XIII), (XIV) or (XV): 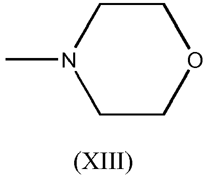 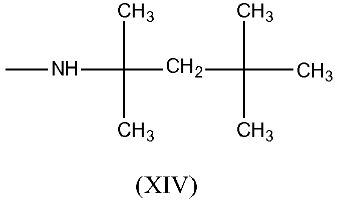 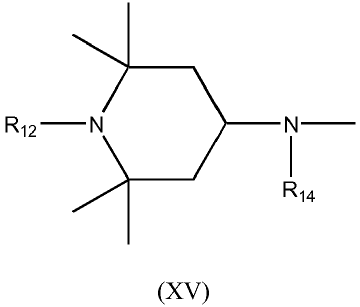 wherein:
   R₁₄ is a straight or branched C₁-C₄ alkyl group;
   R₁₂ is as defined above for the compounds of Formula **R;** 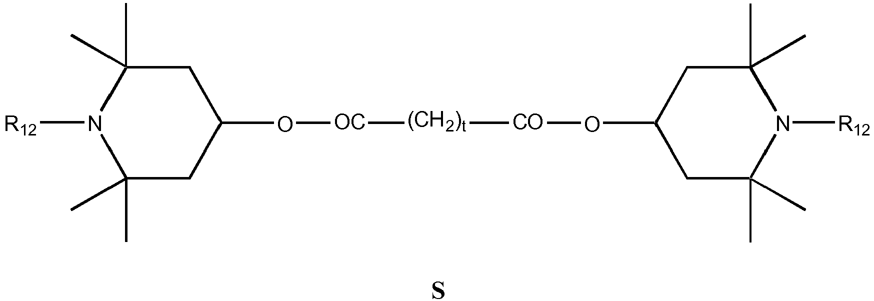
   wherein:
   t is an integer ranging from 2 to 10;
   R₁₂ is as defined above for the compounds of Formula **R;** 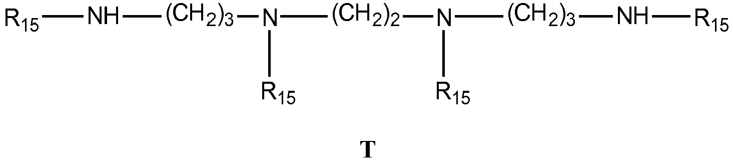
   wherein:
   R₁₅ represents the group of formula (XVI) 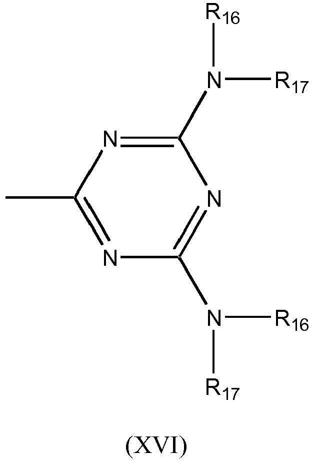 wherein R₁₆ and R₁₇, independently of one another, are selected from the group formed by hydrogen, straight or branched C₁-C₄ alkyl groups and the group of formula (XVII) 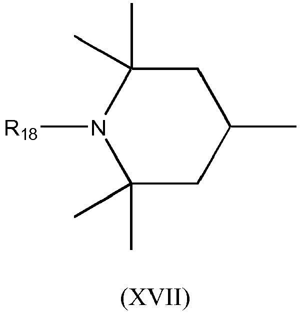 wherein R₁₈ is hydrogen, a straight or branched C₁-C₄ alkyl group or an OR₁₉ group wherein R₁₉ is hydrogen or a straight or branched C₁-C₈ alkyl group; 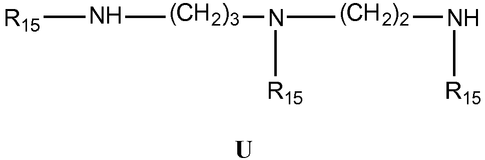 wherein R₁₅ has the meanings defined above for the compounds of formula T.

Said mixtures preferably contain 10% to 90% by weight of the compound of formula (I). More preferably, said mixtures contain 25% to 75% by weight of the compound of formula (I).

Most preferably, said mixtures contain 40% to 60% by weight of the compound of formula (I).

It has been observed that mixtures containing the compounds of formula (I), in particular with n=3-20, R₄=n-butyl and A= -(CH₂)₈-, give polymer materials better stability against photodegradation and the oxidative action of air.

The compounds of formulas P, Q, R, S, T and U and their preparations are known and described in US4477615, EP2632914, US3840494, US3640928, US4331586, EP93693, US4263434, JP57038589 and US6046304.

An example of a compound of formula **P** which can be used in the mixtures according to the invention is the product commercially known by the name of Uvasorb HA88 (3V Sigma S.p.A.).

Another example of a compound of formula **P** which can be used in the mixtures according to the invention is the product commercially known by the name of Uvasorb HA10 (3V Sigma S.p.A.).

An example of a compound of formula **Q** which can be used in the mixtures according to the invention is the product commercially known by the name of Uvasorb HA22 (CAS RN = 65447-77-0).

An example of a compound of formula **R,** wherein W = residue of formula (XIII), which can be used in the mixtures according to the invention is the product commercially known by the name of Cyasorb UV-3346 (CAS RN = 82451-48-7).

An example of a compound of formula **R,** wherein W = residue of formula (XIV), which can be used in the mixtures according to the invention is the product commercially known by the name of Chimassorb 944 (CAS RN = 71878-19-8).

An example of a compound of formula **R,** wherein W = residue of formula (XV), which can be used in the mixtures according to the invention is the product commercially known by the name of Chimassorb 2020 (CAS RN = 192268-64-7).

An example of a compound of formula **S,** wherein R₁₂ = H and t = 8, which can be used in the mixtures according to the invention is the product commercially known by the name of Uvasorb HA77 (CAS RN = 52829-07-9).

An example of a compound of formula **S,** wherein R₁₂ = methyl and t = 8, which can be used in the mixtures according to the invention is the product commercially known by the name of Uvasorb HA29 (CAS RN = 41556-26-7).

An example of a compound of formula **T,** wherein R₁₆ = n-butyl and R₁₇ = residue of formula (XVII) having R₁₈ = methyl, which can be used in the mixtures according to the invention is the product commercially known by the name of Chimassorb 119 (CAS RN = 106990-43-6).

The mixtures of the invention can be obtained by any known method, for example (a) by melting the compounds of formula (I) together with one or more of the compounds of formula P, Q, R, S, T, and/or U, and then grinding or granulating the mixture obtained, (b) dissolving the ingredients in a common solvent and evaporating the solution until dry, or (c) incorporating the compounds separately in the polymer substrate to be stabilised, thereby obtaining the mixture *in situ.*

A further subject of the invention is the use of the compounds of formula (I) alone, mixed together or mixed with at least one of the compounds of formula **P, Q, R, S, T** or **U** as stabilisers for polymers, in particular for polyolefin polymers.

According to the invention, the polymers comprise polyethylene, polypropylene, polystyrene, polybutadiene, polyisoprene and the copolymers thereof, polyvinyl chloride, polivinylidene chloride and the copolymers thereof, polyvinyl acetate and the copolymers thereof, in particular with ethylene; polyesters such as polyethylene terephthalate; polyamides such as Nylon 6 or 6,6; and polyurethanes.

The compounds of the invention and the mixtures of the invention can be incorporated in the polymers by any known method for mixing additives and polymer materials; for example by:
- mixing with the polymer, which can be in the form of a powder or granulate, in a suitable mixer;
- addition in the form of a solution or suspension in a suitable solvent, and subsequent removal of the solvent from the polymer, which may be in the form of a powder, granulate or suspension, after thorough mixing;
- addition to the polymer during its preparation, for example during the last stage of preparation.

The mixtures of the invention can be added together with other types of stabilisers and additives generally used in the art, such as antioxidants based on phenols, amines or phosphites; UV radiation absorbers based on benzophenones or benzotriazoles; nickel-based stabilisers; plasticisers, lubricants, antistatic agents, flame retardants, corrosion inhibitors, metal deactivators and mineral fillers such as titanium dioxide, aluminium oxide and the like.

Examples of said additives are:
1. Antioxidants
   1.1. Alkylated phenols, such as: 2,6-di-tert-butyl-4-methylphenol; 2-tert-butyl-4,6-di-methylphenol; 2,6-di-tert-butyl-4-ethylphenol; 2,6-di-tert-butyl-4-butylphenol; 2,6-di-tert-butyl-4-isobutylphenol; 2,6-di-cyclopentyl-4-methylphenol; 2-(α-methylcyclohexyl)-4,6-di-methylphenol; 2,6-di-octadecyl-4-methylphenol; 2,4,6-tricyclohexylphenol; 2,6-di-tert-butyl-4-methoxymethylphenol; straight and branched nonylphenols, such as 2,6-dinonyl-4-methylphenol; 2,4-di-methyl-6-(1'-methylundecyl)phenol; 2,4-di-methyl-6-(1'-heptadecyl)-phenol and mixtures thereof.
   1.2. Alkyl-thiomethylphenols, such as: 2,4-di-octyl-thiomethyl-6-tert-butylphenol; 2,4-di-octyl-thiomethyl-6-methylphenol; 2,4-di-octyl-thiomethyl-6-ethylphenol; 2,6-di-dodecylthiomethyl-4-nonylphenol.
   1.3. Hydroquinones and alkylated hydroquinones, such as: 2,6-di-tert-butyl-4-methoxyphenol; 2,5-di-tert-butyl-hydroquinone; 2,5-di-tert-amyl-hydroquinone; 2,6-diphenyl-4-octadecyloxyphenol; 2,6-di-tert-butyl-hydroquinone; 2,5-di-tert-butyl-4-hydroxyanisole; 3,5-di-tert-butyl-4-hydroxyanisole; 3,5-di-tert-butyl-4-hydroxyphenyl stearate; bis(3,5-di-tert-butyl-4-hydroxyphenyl)adipate.
   1.4. Tocopherols, such as α-tocopherol; γ-tocopherol; β-tocopherol; δ-tocopherol and mixtures thereof (vitamin E).
   1.5. Hydroxylated thiodiphenyl ethers, such as: 2,2'-thiobis(6-tert-butyl-4-methylphenol); 2,2'-thiobis(4-octylphenol); 4,4'-thiobis(6-tert-butyl-3-methylphenol); 4,4'-thiobis(6-tert-butyl-2-methylphenol); 4,4'-bis(2,6-di-methyl-4-hydroxyphenyl)-disulphide.
   1.6. Alkylidene bisphenols, such as: 2,2'-methylenebis(6-tert-butyl-4-methylphenol); 2,2'-methylenebis(6-tert-butyl-4-ethylphenol); 2,2'-methylenebis(4-methyl-6-(α-methylcyclohexyl)phenol); 2,2'-methylenebis(4-methyl-6-cyclohexylphenol); 2,2'-methylenebis(6-nonyl-4-methylphenol); 2,2'-methylenebis-(4,6-di-tert-butylphenol); 2,2'-ethylidenebis-(4,6-di-tert-butylphenol); 2,2'-ethylidenebis(6-tert-butyl-4-isobutylphenol); 2,2'-methylenebis(6-(α-methylbenzyl)-4-nonylphenol); 2,2'-methylenebis(6-(α-αdimethylbenzyl)-4-nonylphenol); 4,4'-methylenebis-(2,6-di-tert-butylphenol); 4,4'-methylenebis(6-tert-butyl-2-methyl-phenol); 1,1-bis(5-tert-butyl-4-hydroxy-2-methylphenyl)-butane; 2,6-bis(3-tert-butyl-5-methyl-2-hydroxybenzyl)-4-methylphenol; 1,1,3-tris-(5-tert-butyl-4-hydroxy-2-methylphenyl)-butane; 1,1-bis(5-tert-butyl-4-hydroxy-2-methylphenyl)-3-n-dodecyl-mercaptobutane; ethylene glycol bis(3,3-bis(3'-tert-butyl-4'-hydroxyphenyl)-butyrate); bis(2-(3'-tert-butyl-2'-hydroxy-5'-methylbenzyl)-6-tert-butyl-4-methylphenyl)-terephthalate; bis(3-tert-butyl-4-hydroxy-5-methylphenyl)-dicyclopentadiene; 1,1-bis-(3,5-dimethyl-2-hydroxyphenyl)butane; 2,2,bis-(3,5-di-tert-butyl-4-hydroxyphenyl)propane; 2,2-bis(5-tert-butyl-4-hydroxy-2-methylphenyl)-4-n-dodecyl-mercaptobutane; 1,1,5,5-tetra-(5-tert-butyl-4-hydroxy-2-methylphenyl)-pentane.
   1.7. O-, N- and S-benzyl derivatives, such as: 3,5,3',5'-tetra-tert-butyl-4-4'-dihydroxydibenzyl ether; octadecyl-4-hydroxy-3,5-dimethylbenzyl-mercapto acetate; tridecyl-4-hydroxy-3,5-di-tert-butyl-benzylmercapto acetate; tri(3,5-di-tert-butyl-4-hydroxybenzyl)amine; bis(4-tert-butyl-3-hydroxy-2,6-dimethylbenzyl)dithioterephthalate; bis(3,5-di-tert-butyl-4-hydroxybenzyl)disulphide; isooctyl-3,5-di-tert-butyl-4-hydroxybenzylmercaptoacetate.
   1.8. Malonates containing the hydroxybenzyl group, such as: dioctadecyl-2,2-bis-(3,5-di-tert-butyl-2-hydroxybenzyl)malonate; dioctadecyl-2-(3-tert-butyl-4-hydroxy-5-methylbenzyl)malonate; di-dodecylmercaptoethyl-2,2-bis-(3,5-di-tert-butyl-4-hydroxybenzyl)malonate; bis(4-(1,1,3,3-tetramethylbutyl)-phenyl)-2,2-bis-(3,5-di-tert-butyl-4- hydroxybenzyl)malonate.
   1.9. Aromatic hydroxybenzyl compounds, such as: 1,3,5-tris-(3,5-di-tert-butyl-4-hydroxybenzyl)-2,4,6-trimethylbenzene; 1,4-bis-(3,5-di-tert-butyl-4-hydroxybenzyl)-2,3,5,6-tetramethylbenzene; 2,4,6-tris-(3,5-di-tert-butyl-4-hydroxybenzyl)-phenol.
   1.10. Triazine derivatives, such as: 2,4-bis(octylmercapto)-6-(3,5-di-tert-butyl-4-hydroxyanilino)-1,3,5-triazine; 2-octylmercapto-4-6-bis(3,5-di-tert-butyl-4-hydroxyanilino)-1,3,5-triazine; 2-octylmercapto-4-6-bis(3,5-di-tert-butyl-4-hydroxyphenoxy)-1,3,5-triazine; 2,4,6-tris(3,5-di-tert-butyl-4-hydroxy-phenoxy)-1,3,5-triazine; 1,3,5-tris-(3,5-di-tert-butyl-4-hydroxybenzyl)-isocyanurate; 1,3,5-tris-(4-tert-butyl-3-hydroxy-2,6-dimethylbenzyl)isocyanurate; 2,4,6-tris(3,5-di-tert-butyl-4-hydroxyphenylethyl)-1,3,5-triazine; 1,3,5-tris-(3,5-di-tert-butyl-4-hydroxyphenylpropionyl)-hexahydro-1,3,5-triazine; 1,3,5-tris-(3,5-dicyclohexyl-4-hydroxybenzyl)isocyanurate.
   1.11. Benzylphosphonates, such as: dimethyl-2,5-di-tert-butyl-4-hydroxybenzylphosphonate; diethyl-3,5-di-tert-butyl-4-hydroxybenzylphosphonate; dioctadecyl-3,5-di-tert-butyl-4-hydroxybenzylphosphonate; dioctadecyl-5-tert-butyl-4-hydroxy-3-methylbenzylphosphonate; the calcium salt of 3,5-di-tert-butyl-4-hydroxybenzylphosphonic acid monoethyl ester.
   1.12. Acylaminophenols, such as lauric acid 4-hydroxyanilide, stearic acid 4-hydroxyanilide and octyl N-(3,5-di-tert-butyl-4-hydroxyphenyl)carbamate.
   1.13. Esters of β-(3,5-di-tert-butyl-4-hydroxyphenyl)propionic acid with mono- or polyhydric alcohols, such as: methanol, ethanol, n-octanol, iso-octanol and octadecanol; 1,6-hexanediol; 1,9-nonanediol; ethylene glycol; 1,2-propanediol; neopentyl glycol; thiodiethylene glycol; diethylene glycol; triethylene glycol; pentaerythritol; tri-(hydroxyethyl)isocyanurate; N,N' -bis(hydroxyethyl)oxamide; 3-thioundecanol; 3-thiopentadecanol; trimethylhexanediol; trimethylolpropane; 4-hydroxymethyl-1-phospho-2,6,7-trioxabicyclo(2,2,2)octane.
   1.14. Esters of β-(5-tert-butyl-4-hydroxy-3-methylphenyl)propionic acid with mono- or polyhydric alcohols, such as: methanol, ethanol, n-octanol, iso-octanol and octadecanol; 1,6-hexanediol; 1,9-nonanediol; ethylene glycol; 1,2-propanediol; neopentyl glycol; thiodiethylene glycol; diethylene glycol; triethylene glycol; pentaerythritol; tri-(hydroxyethyl)isocyanurate; N,N' -bis(hydroxyethyl)oxamide; 3-thioundecanol; 3-thiopentadecanol; trimethylhexanediol; trimethylolpropane; 4-hydroxymethyl-1-phospho-2,6,7-trioxabicyclo(2,2,2)octane.
   1.15. Esters of β-(3,5-dicyclohexyl-4-hydroxyphenyl)propionic acid with mono- or polyhydric alcohols, such as: methanol, ethanol, n-octanol, iso-octanol and octadecanol; 1,6-hexanediol; 1,9-nonanediol; ethylene glycol; 1,2-propanediol; neopentyl glycol; thiodiethylene glycol; diethylene glycol; triethylene glycol; pentaerythritol; tri-(hydroxyethyl)isocyanurate; N,N' -bis(hydroxyethyl)oxamide; 3-thioundecanol; 3-thiopentadecanol; trimethylhexanediol; trimethylolpropane; 4-hydroxymethyl-1-phospho-2,6,7-trioxabicyclo(2,2,2)octane.
   1.16. Esters of 3,5-di-tert-butyl-4-hydroxyphenyl acetic acid with mono- or polyhydric alcohols, such as: methanol, ethanol, n-octanol, iso-octanol and octadecanol; 1,6-hexanediol; 1,9-nonanediol; ethylene glycol; 1,2-propanediol; neopentyl glycol; thiodiethylene glycol; diethylene glycol; triethylene glycol; pentaerythritol; tri-(hydroxyethyl)isocyanurate; N,N' -bis(hydroxyethyl)oxamide; 3-thioundecanol; 3-thiopentadecanol; trimethylhexanediol; trimethylolpropane; 4-hydroxymethyl-1-phospho-2,6,7-trioxabicyclo(2,2,2)octane.
   1.17. Amides of β-(3,5-di-tert-butyl-4-hydroxyphenyl)propionic acid, such as: N,N'-bis-(3,5-di-tert-butyl-4-hydroxyphenylpropionyl)-hexamethylenediamide; N,N'-bis-(3,5-di-tert-butyl-4-hydroxyphenylpropionyl)-trimethylenediamide; N,N'-bis-(3,5-di-tert-butyl-4-hydroxyphenylpropionyl)-hydrazide; N,N' -bis-(2-(3 -(3,5 -di-tert-butyl-4-hydroxyphenyl)propionyloxy)ethyl)-oxamide.
   1.18. Ascorbic acid (vitamin C).
   1.19. Amine antioxidants, such as: N,N'-di-isopropyl-p-phenylenediamine; N,N'-di-sec-butyl-p-phenylenediamine; N,N'-bis(1,4-dimethylpentyl)-p-phenylenediamine; N,N' -bis(1 -ethyl-3 -methylpentyl)-p-phenylenediamine; N,N'-bis(1-methylheptyl)-p-phenylenediamine; N,N'-dicyclohexyl-p-phenylenediamine; N,N'-diphenyl-p-phenylenediamine; N,N'-bis-(2-naphthyl)-p-phenylenediamine; N-isopropyl-N' -phenyl-p-phenylenediamine; N-(1,3-dimethylbutyl)-N' -phenyl-p-phenylenediamine; N-(1-methylheptyl)-N'-phenyl-p-phenylenediamine; N-cyclohexyl-N'-phenyl-p-phenylenediamine, 4-(p-toluen-sulphamoyl)diphenylamine; N,N'-dimethyl-N,N'-di-sec-butyl-p-phenylenediamine; diphenylamine; N-allyl-diphenylamine; 4-isopropoxydiphenylamine, N-phenyl-1-naphthylamine; N-(4-tert-octylphenyl)-1-naphthylamine; N-phenyl-2-naphthylamine; p,p'-di-tert-octyl-diphenylamine; 4-n-butyl-aminophenol; 4-butyryl-aminophenol; 4-nonanoyl-aminophenol; 4-dodecanoyl-aminophenol; 4-octadecanoyl-aminophenol; bis(4-methoxyphenyl)amine; 2,6-di-tert-butyl-4-dimethylaminomethylphenol; 2,4'-diaminodiphenylmethane; 4,4' -diaminodiphenyl-methane; N,N,N',N' -tetramethyl-4,4' -diaminodiphenylmethane; 1,2-bis-((2-methylphenyl)-amino)ethane; 1,2-bis-(phenylamine)propane; o-tolyl-biguanide; bis-(4-(1',3'-dimethylbutyl)phenyl)amine; tert-octyl-N-phenyl-1-naphthylamine; mixtures of dialkylated tert-butyl/tert-octyl-diphenylamines; mixtures of mono- and di-alkyl nonyldiphenylamines; mixtures of mono- and di-alkyl dodecyldiphenylamines; mixtures of mono- and di-alkyl isopropyl/isohexyldiphenylamines; mixtures of mono- and di-alkyl tert-butyldiphenylamines; 2,3-dihydro-3,3-dimethyl-4H-1,4-benzothiazine; phenothiazine; mixtures of mono- and dialkyl tert-butyl/tert-octylphenothiazines, mixtures of mono- and dialkyl tert-octyl-phenothiazines; N-allylphenothiazine; N,N,N',N'-tetraphenyl-1,4-diamino-2-butene; N,N'-bis-(2,2,6,6-tetramethyl-piperidyl-4-hexamethylenediamine; bis(2,2,6,6-tetramethylpiperid-4-yl)sebacate; 2,2,6,6-tetramethylpiperid-4-one; 2,2,6,6-tetramethylpiperid-4-ol.
2. UV radiation absorbers and light stabilisers
   2.1. 2-(2'-hydroxyphenyl)benzotriazoles, such as: 2-(2'-hydroxy-5-methylphenyl)benzotriazole; 2-(3',5'-di-tert-butyl-2'-hydroxyphenyl)benzotriazole; 2-(5'-tert-butyl-2'-hydroxyphenyl)benzotriazole; 2-(2'-hydroxy-5'-(1,1,3,3-tetramethylbutyl)-enyl)benzotriazole; 2-(3',5'-di-tert-butyl-2'-hydroxyphenyl)-5-chloro-benzotriazole; 2-(3 '-tert-butyl-2'-hydroxy-5 '-methylphenyl)-5-chloro-benzotriazole; 2-(3'-sec-butyl-5'-tert-butyl-2' -hydroxyphenyl)-benzotriazo le; 2-(2'-hydroxy-4'-octyloxyphenyl)benzotriazole; 2-(3',5'-di-tert-amyl-2'-hydroxyphenyl)benzotriazole; 2-(3',5'-bis-(α,α-dimethylbenzyl)-2'-hydroxyphenyl)benzotriazole; 2-(3'-tert-butyl-5'-(2-(2-ethylhexyloxy)-carbonylethyl)-2 '-hydroxyphenyl)-5-chloro-benzotriazole; 2-(3'-tert-butyl-2'-hydroxy-5'-(2-octyloxycarbonylethyl)phenyl)-5-chloro-benzotriazole; 2-(3'-tert-butyl-2'-hydroxy-5'-(2-methoxycarbonylethyl)phenyl)-5-chloro-benzotriazole; 2-(3'-tert-butyl-2'-hydroxy-5'-(2-methoxycarbonylethyl)phenyl)-benzotriazole; 2-(3 '-tert-butyl-2 '-hydroxy-5 '-(2-octyloxycarbonylethyl)phenyl)-benzotriazole; 2-(3'-tert-butyl-5'-(2-(2-ethylhexyloxy)-carbonylethyl)-2'-hydroxyphenyl)-benzotriazole; 2-(3'-dodecyl-2'-hydroxy-5 '-methylphenyl)-benzotriazole; 2-(3'-tert-butyl-2'-hydroxy-5'-(2-isooctyloxy-carbonylethyl)-phenylbenzotriazole; 2,2'-methylene-bis-(4-(1,1,3,3-tetramethylbutyl)-6-benzotriazol-2-ylphenol); the product of transesterification of 2-(3'-tert-butyl-5'-(2-methoxycarbonylethyl)-2'-hydroxyphenyl)-2H-benzotriazole with polyethylene glycol 300; (R-CH₂-CH₂-COO-CH₂-CH₂-)₂- wherein R can be: 3'-tert-butyl-4'-hydroxy-5'-2H-benzotriazol-2-ylphenyl; 2-(2'-hydroxy-3'-(α,α-dimethylbenzyl)-5'-(1,1,3,3-tetramethylbutyl)-phenyl)benzotriazole; 2-(2'-hydroxy-3'-(1,1,3,3-tetramethylbutyl)-5'-(α,α-dimethylbenzyl)phenyl)benzotriazole.
   2.2. 2-hydroxybenzophenones, such as the 4-hydroxy; 4-methoxy; 4-octyloxy; 4-decyloxy; 4-dodecyloxy; 4-benzyloxy; 4,2',4'-tri-hydroxy and 2'-hydroxy-4,4'-dimethoxy derivatives.
   2.3. Esters of substituted and non-substituted benzoic acids, such as: 4-tertbutyl-phenyl-salicylate; phenyl salicylate; octylphenyl salicylate; dibenzoyl resorcinol; bis-(4-tert-butyl-benzoyl)-resorcinol; benzoyl resorcinol; 2,4-di-tert-butylphenyl 3,5-di-tert-butyl-4-hydroxybenzoate; hexadecyl 3,5-di-tert-butyl-4-hydroxybenzoate; octadecyl 3,5-di-tert-butyl-4-hydroxy-benzoate; 2-methyl-4,6-di-tert-butylphenyl 3,5-di-tert-butyl-4-hydroxybenzoate:
   2.4. Acrylates, such as: ethyl α-cyano-β,β-diphenylacrylate; isooctyl α-cyano-β,β-diphenylacrylate; methyl α-carbomethoxycinnamate; methyl α-cyano-β-methyl-p-methoxycinnamate; butyl α-cyano-β-methyl-p-methoxy-cinnamate; methyl α -carbomethoxy-p-methoxycinnamate and N-(β-carbomethoxy-β-cyanovinyl)-2-methylindoline.
   2.5. Nickel derivatives, such as: 1:1 or 1:2 complexes of nickel with 2,2'-thio-bis-(4-(1,1,3,3-tetramethylbutyl)phenol, with or without binders such as n-butylamine, triethanolamine or N-cyclohexyldiethanolamine; nickel dibutylthiodicarbamate; nickel salts of 4-hydroxy-3,5-di-tert-butylbenzylphosphonic acid mono-alkyl esters (such as methyl or ethyl esters); nickel complexes with keto-oxime, for example with 2-hydroxy-4-methylphenyl undecyl-keto-oxime; nickel complexes with 1-phenyl-4-lauroyl-5-hydroxy-pyrazole, with or without additional binders.
   2.6. Oxamides, such as: 4,4'-dioctyloxy-oxalanilide; 2,2'-diethoxy-oxalanilide; 2,2'-dioctyloxy-5,5'-di-tert-butyl-oxalanilide; 2,2'-didodecyloxy-5,5'-di-tert-butyl-oxalanilide; 2-ethoxy-2 '-ethyloxy-oxalanilide; N,N'-bis(3-dimethylaminopropyl)-oxalanilide; 2-ethoxy-2'-ethyl-5,4'-di-tert-butyl-oxalanilide; mixtures of disubstituted o- and p-methoxy oxalanilides and mixtures of disubstituted o- and p-ethoxy oxalanilides.
   2.7. 2-(2-hydroxyphenyl)-1,3,5-triazines, such as: 2,4,6-tris(2-hydroxy-4-octyloxyphenyl)-1,3,5-triazine; 2-(2-hydroxy-4-octyloxyphenyl)-4,6-bis(2,4-dimethylphenyl)-1,3,5-triazine; 2,4-bis(2-hydroxy-4-propyloxyphenyl)-6-(2,4-dimethylphenyl)-1,3,5-triazine; 2-(2-hydroxy-4-octyloxyphenyl)-4,6-bis-(4-methylphenyl)-1,3,5-triazine; 2-(2-hydroxy-4-dodecyloxyphenyl)-4,6-bis-(2-4-dimethylphenyl)-1,3,5-triazine; 2-(2-hydroxy-4-tridecyloxyphenyl)-4,6-bis-(2-4-dimethyl-phenyl)-1,3,5-triazine; 2-(2-hydroxy-4-(2-hydroxy-3-butyloxy-propoxy)phenyl)-4,6-bis(2,4-dimethyl)-1,3,5-triazine; 2-(2-hydroxy-4-(2-hydroxy-3-octyloxy-propoxy)-phenyl)-4,6-bis(2,4-dimethyl)-1,3,5-triazine; 2-(4-(dodecyloxy/tridecyloxy-2-hydroxypropoxy)-2-hydroxyphenyl)-4,6-bis(2,4-dimethylphenyl)-1,3,5-triazine; 2-(2-hydroxy-4-(2-hydroxy-3-dodecyloxy-propoxy)phenyl)-4,6-bis(2,4-dimethylphenyl)-1,3,5-triazine; 2-(2-hydroxy-4-hexyloxy)phenyl-4,6-diphenyl-1,3,5-triazine; 2-(2-hydroxy-4-methoxyphenyl)-4,6-diphenyl-1,3,5-triazine; 2,4,6-tris(2-hydroxy-4-(3-butoxy-2-hydroxy-propoxy)phenyl-1,3,5-triazine; 2-(2-hydroxyphenyl)-4-(4-methoxyphenyl)-6-phenyl-1,3,5-triazine; 2-(2-hydroxy-4-(3-(2-ethylhexyl-1-oxy)-2-hydroxypropyloxy)phenyl)-4,6-bis(2,4-dimethylphenyl)-1,3,5-triazine.
   2.8. Triazine derivatives, such as: diethylhexyl butamido triazone, ethylhexyl triazone, 2,4-bis-[4-[5-(1,1-dimethyl-propyl)benzoxazol-2-yl]phenylimino]-6-[(2-ethylhexyl)imino] -1,3,5 -triazine, tris-biphenyltriazine, bis-ethylhexyloxyphenol-methoxyphenyl-triazine.
3. Metal deactivators, such as: N,N'-diphenyloxamide; N-salicylal-N'-salicylol-hydrazine; N,N'-bis(salicylol)hydrazine; N,N'-bis-(3,5-di-tert-butyl-4-hydroxyphenylpropionyl)-hydrazine; 3-salicylolamino-1,2,4-triazole; bis(benzylidene)-oxalyl dihydrazide; oxalanilide; isophthaloyl dihydrazide; sebacoyl bisphenylhydrazide; N,N'-diacetyladipoyl dihydrazide; N,N'-bis(salicyloyl)oxalyl dihydrazide; N,N' -bis(salicyloyl)thiopropionyl dihydrazide.
4. Phosphites and phosphonites, such as: triphenyl phosphite; diphenyl alkyl phosphites; phenyl dialkyl phosphites; tris(nonylphenyl)phosphite; trilauryl phosphite; trioctadecyl phosphite; distearyl pentaerythritol diphosphite; tris(2,4-di-tert-butyl-phenyl) phosphite; diisodecyl pentaerythritol diphosphite; bis(2,4-di-tert-butylphenyl) phosphite; diisodecyl pentaerythritol diphosphite; bis(2,4-di-tert-butylphenyl) pentaerythritol diphosphite; bis(2,6-di-tert-butyl-4-methylphenyl)-pentaerythritol diphosphite; diisodecyloxy-pentaerythritol diphosphite; bis-(2,4-di-tert-butyl-6-methylphenyl)pentaerythritol diphosphite; bis(2,4,6-tris(tert-butylphenyl) pentaerythritol diphosphite; tristearyl sorbitol triphosphite; bis(2,4-di-tert-butyl-6-methylphenyl) methyl phosphite; bis(2,4-di-tert-butyl-6-methylphenyl) ethyl phosphite; 2,2',2"-nitrilo(triethyltris(3,3',5,5'-tetra-tert-butyl-1,1'-biphenyl-2,2'-idyl) phosphite); 2-ethylhexyl (3,3',5,5'-tetra-tert-butyl-1,1'-biphenyl-2,2'-idyl) phosphite); tetra(2,4-di-tert-butylphenyl) 4-4'-biphenylene diphosphonite.
5. Hydroxylamines, such as: N,N-dibenzylhydroxylamine; N,N-diethylhydroxylamine; N,N-dioctylhydroxylamine; N,N-dilaurylhydroxylamine; N,N-ditetradecylhydroxylamine; N,N-dihexadecyl-hydroxylamine; N,N-dioctadecylhydroxylamine; N-hexadecyl-N-octadecylhydroxylamine; N-heptadecyl-N-octadecylhydroxylamine; N,N-dialkylhydroxylamines derived from hydrogenated tallow amines.
6. Nitrones, such as: N-benzyl-alpha-phenyl-nitrone; N-ethyl-alpha-methyl-nitrone; N-octyl-alpha-heptyl-nitrone; N-lauryl-alpha-undecyl-nitrone; N-tetradecyl-alpha-tridecyl-nitrone; N-hexadecyl-alpha-pentadecyl-nitrone; N-octadecyl-alpha-pentadecyl-nitrone; N-heptadecyl-alpha-heptadecyl-nitrone; N-octadecyl-alpha-hexadecyl-nitrone; nitrones derived from N,N-dialkylhydroxylamines obtained from hydrogenated tallow amines.
7. Thiosynergistic derivatives such as dilauryl thiodipropionate or stearyl thiodipropionate.
8. Antiperoxide agents, such as: esters of thiodipropionic acid with lauryl, stearyl, myristyl or tridecyl alcohols; mercaptobenzimidazole or 2-mercapto-benzoimidazole zinc salt; zinc dibutyldithiocarbamate; dioctadecyl disulphide; pentaerythritol tetrakis(β-dodecylmercapto)propionate.
9. Polyamide stabilisers, such as: copper salts in combination with iodides and/or phosphorated compounds and bivalent manganese salts.
10. Basic co-stabilisers, such as: melamine; polyvinylpyrrolidone; dicyandiamide; triallylcyanurate; urea derivatives; hydrazine derivatives; amines; polyamides; polyurethanes; alkali metal and alkaline earth metal salts of higher fatty acids such as calcium stearate and zinc stearate; magnesium behenate; magnesium stearate; sodium ricinoleate; potassium palmitate; pyrocatechol antimony or zinc salts.
11. Nucleating agents, such as: inorganic substances such as talc; metal oxides such as titanium dioxide or magnesium oxide; phosphates, carbonates or sulphates or alkali earth metal salts; organic compounds such as mono- or polycarboxylic acids and the salts thereof, such as 4-tert-butylbenzoic acid, adipic acid, diphenylacetic acid and sodium succinate; sodium benzoate; polymer compounds such as anionic copolymers.
12. Benzofuranones and indolinones, such as those described in US 4,325,863; US 4,338,244; US 5,175,312; US 5,216,052; US 5,252,643; DE 4316611; DE 4316622; DE 4316876; EP 0589839; and EP 0591102; 3-(4-(2-acetoethoxy)phenyl)-5,7-di-tert-butyl-benzofuran-2-one; 5,7-di-tert-butyl-3-(4-(2-stearoyloxyethoxy)phenyl)-benzofuran-2-one; 3,3'-bis(5,7-di-tert-butyl-3-(4-(2-hydroxyethoxy)phenyl)benzofuran-2-one); 5,7-di-tert-butyl-3-(4-ethoxyphenyl)-benzofuran-2-one; 3-(4-acetoxy-3,5-dimethylphenyl)-5,7-di-tert-butyl-benzofuran-2-one; 3-(3,5-dimethyl-4-pivaloyloxyphenyl)-5,7-di-tert-butyl-benzofuran-2-one; 3-(2,3-di-methylphenyl)-5,7-di-tert-butyl-benzofuran-2-one.
13. Fillers and reinforcing agents, such as: calcium carbonate; silicates; fibreglass; asbestos; talc; kaolin; mica; barium sulphate; metal oxides and hydroxides, titanium dioxide in its various forms, and carbon black; graphite; wood flour, fibre flour or other natural products; synthetic fibres.
14. Other additives, such as: plasticisers, lubricants, emulsifiers, pigments and rheology modifiers; catalysts; flow control agents; optical brighteners; flame retardants; antistatic agents and swelling agents.

The amount of mixtures according to the invention required for effective stabilisation of the polymer depends on a number of factors, such as the type and characteristics of the polymer, its intended use, the intensity of the radiation, and the duration of the likely exposure.

An amount of mixture ranging from 0.01 to 5% by weight of the polymer, preferably 0.1 to 1.0%, is usually sufficient.

The examples below illustrate the invention in detail.

### Example 1: Preparation of 2,2'-[[4,6-bis[butyl(2,2,6,6-tetramethyl-4-piperidinyl)amino]-1,3,5-triazin-2-yl]imino]bis-ethanol corresponding to the molecule of formula III with R₃=R₄=butyl; R₅ = R₆ = H; D = -(CH₂)₂-

300 g of xylene, 2.13 g of sodium hydrogen carbonate and 55.5 g of cyanuryl chloride are loaded into a 1 L flask under nitrogen. 72.0 g of N-butyl-2,2,6,6-tetramethylpiperidin-4-amine and 97.4 g of 18.5% aqueous Na₂CO₃ are added to the stirred mixture in sequence, maintaining the temperature at between 50 and 80°C, followed by a further 55.5 g of N-butyl-2,2,6,6-tetramethylpiperidin-4-amine and 97.4 g of 18.5% aqueous Na₂CO₃. The reaction is completed by maintaining a slight reflux for two hours, and the lower aqueous phase is then discharged. Maintaining the temperature at about 80°C, 43.5 g of diethanolamine and 96.1 g of 18.5% aqueous Na₂CO₃ are added. The mixture is heated to reflux, removing the water with a Dean Stark apparatus. The mixture is kept under stirring at the same temperature for 6 h, and 150 g of xylene and 130 g of demineralised H₂O are then added. The reaction mixture is stirred at 90-95°C for 30 minutes, and the underlying aqueous phase is then discharged. After a further aqueous wash, the solvent and residual water are removed under vacuum. 182.1 g of product is obtained by solidifying the molten mass. UPLC-MS analysis gave an assay value = 99.69%.

### Example 2: Preparation of HALS1, corresponding to a polymer of formula I with A = -(CH₂)₈- ; R₁ = R₂ = methyl; B = a group of formula II with R₃ = R₄ = butyl; R₅ = R₆ = H; D = -(CH₂)₂- obtainable by reacting 2,2'-[[4,6-bis[butyl(2,2,6,6-tetramethyl-4-piperidinyl)-amino]-1,3,5-triazin-2-yl]imino]bis-ethanol with decanedioic acid 1,10-dimethyl ester.

11.99 g (52.1 mmols) of dimethyl sebacate and 30.0 g (49.6 mmols) of triazine intermediate corresponding to the molecule of formula III in example 1 are loaded into a 250 mL flask under nitrogen. The temperature is increased to 140°C, and 0.05 g of titanium (IV) isopropoxide is added to the molten mass. The temperature is gradually increased to 160°C, and the reaction is completed by removing under vacuum the methanol formed and the excess unreacted dimethyl sebacate. 100 g of xylene is added and the catalyst is removed by hot aqueous washing, then azeotropically dried and clarified by hot filtration. After removal of the xylene under vacuum, the molten mass is cooled to obtain 34.6 g of the HALS1 product in the form of pale yellow flakes. A Mw = 13849 g/mole was measured with GPC analysis.

### Example 3: Preparation of 2,2'-[[4,6-bis[butyl(1,2,2,6,6-pentamethyl-4-piperidinyl)amino]-1,3,5-triazin-2-yl]imino]bis-ethanol corresponding to the molecule of formula III with R₃ = R₄= butyl; R₅ = R₆ = methyl; D = -(CH₂)₂-

300 g of xylene, 2.13 g of NaHCO₃ and 55.5 g of cyanuryl chloride are loaded into a 1 L flask under nitrogen. 72.0 g of N-butyl-2,2,6,6-tetramethylpiperidin-4-amine and 97.4 g of 18.5% aqueous Na₂CO₃ are added to the stirred mixture in sequence, maintaining the temperature at between 50 and 80°C, followed by a further 55.5 g of N-butyl-2,2,6,6-tetramethylpiperidin-4-amine and 97.4 g of 18.5% aqueous Na₂CO₃. The reaction is completed by maintaining a slight reflux for two hours, and the lower aqueous phase is then discharged. Maintaining the temperature at about 80°C, 90.2 g of 30% aqueous formaldehyde and 46.1 g of 90% formic acid are added in about 2 hours. The reaction is completed at 85°C, then the mixture is cooled to 60°C and 150 g of 10% aqueous NaOH is added. After mixing, the lower aqueous phase is discharged and the organic phase is washed with demineralised H₂O.

Maintaining the temperature at about 80°C, 42.8 g of diethanolamine and 96.1 g of 18.5% aqueous Na₂CO₃ are added. The mixture is heated to reflux, removing the water with a Dean Stark apparatus. The mixture is maintained under stirring at the same temperature for 6 h, then 130 g of demineralised H₂O is added. The reaction mixture is stirred at 90-95°C for 30 minutes, and the underlying aqueous phase is then discharged. After a further aqueous wash, the solvent and residual water are removed under vacuum. 185.0 g of product is obtained by solidification of the molten mass. UPLC-MS analysis gave an assay value = 99.52%

### Example 4: Preparation of HALS2, corresponding to a polymer of formula I with A = -(CH₂)₈- ; R1 = R2 = methyl; B = group of formula II with R₃ = R₄ = butyl; R₅ = R₆ = methyl; D = -(CH₂)₂- obtainable by reacting 2,2'-[[4,6-bis[butyl(1,2,2,6,6-pentamethyl-4-piperidinyl)-amino]-1,3,5-triazin-2-yl]imino]bis-ethanol with decanedioic acid 1,10-dimethyl ester.

11.49 g (49.9 mmols) of dimethyl sebacate and 30.07 g (47.5 mmols) of triazine intermediate corresponding to the molecule of formula III in example 3 are loaded into a 250 mL flask under nitrogen. The temperature is increased to 140°C and 0.05 g of titanium (IV) isopropoxide is added to the molten mass. The temperature is gradually increased to 160°C, and the reaction is completed by removing under vacuum the methanol formed and the excess unreacted dimethyl sebacate. 100 g of xylene is added and the catalyst is removed by hot aqueous washing, then azeotropically dried and clarified by hot filtration. After removal of the xylene under vacuum, the molten mass is cooled to obtain 36.0 g of the HALS2 product in the form of pale yellow flakes.

A Mw = 12178 g/mole was measured with GPC analysis.

### Application examples

All amounts are expressed in weight unless otherwise stated.

### Example 5: Light stabilisation of polypropylene fibre

4 samples were prepared by the following procedure:
1000 parts by weight of unstabilised polypropylene homopolymer (fluidity index about 10-12 g / 10 min at 230°C - 2.16 kP) were mixed in a laboratory mixer with 1.0 parts by weight of calcium stearate, 0.50 parts by weight of tris-(2,4-di-tert-butyl-phenyl) phosphite, 0.50 parts by weight of 1,3,5-tris-(3,5-di-tert-butyl-4-hydroxybenzyl) isocyanurate, to three of which 1.5 parts by weight of stabilisers HALS1, HALS2 and HALS3 respectively were added, wherein HALS3 is a hindered polyester polymer amine containing the hindered amino groups inserted directly into the polymer chain identified by CAS RN = 65447-77-0.

The dry mixtures thus obtained were extruded in a laboratory extruder at 230°C, and granulated after cooling of the extrusion.

The granulates were then converted to a film with a thickness of 100 microns, using a laboratory press by compression moulding at 210°C.

Specimens obtained from the various films were exposed in a Weather-Ometer Mod. Ci35A, according to ISO 4892 (T black panel 63 ± 2°C, dry cycle). Specimens were taken periodically to undergo a Carbonyl Index test, using the FT-IR spectrophotometry technique. The increase in the value of the Carbonyl Index provides an indication of the state of photooxidative degradation of the material, and is correlatable with the loss of mechanical properties resulting from the photooxidation of the material.

The parameter used to compare the light resistance of the samples was t_{0.10}, defined as "exposure time in WOM, expressed in hours, on reaching a Carbonyl Index value of 0.10".

The experimental results obtained are summarised in the Table.

**TABLE. Light stability of 100 micron PP film**

| ***Stabilisation*** | ***t_{0.10}*** |
|---|---|
| Without stabiliser | 700 |
| 0.15% HALS1 | 1730 |
| 0.15% HALS2 | 1800 |
| 0.15% HALS 3 | 1250 |

The results of this example clearly demonstrate that the hindered polymer amines according to the present invention, containing the hindered amino groups in the mobile pendants of the polymer structure, are much more effective than those wherein the hindered amino group is inserted directly into the polymer chain.

## Claims

1. Compounds of general formula (I): 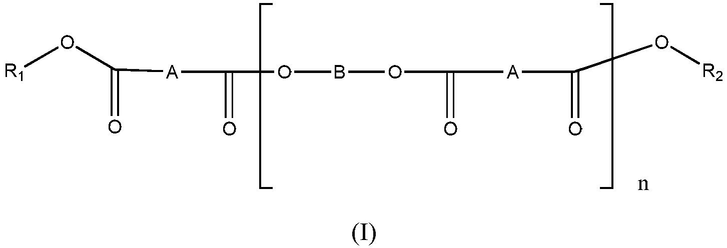 wherein:
n is an integer ranging from 2 to 100;
R₁ and R₂ are hydrogen or straight or branched, saturated or unsaturated C₁-C₁₈ alkyl groups or aromatic groups;
A is a C₁-C₂₀ alkylene or C₃-C₁₀ cycloalkylene group optionally containing one or more unsaturations, or an aromatic group;
and B is a group of formula (II): 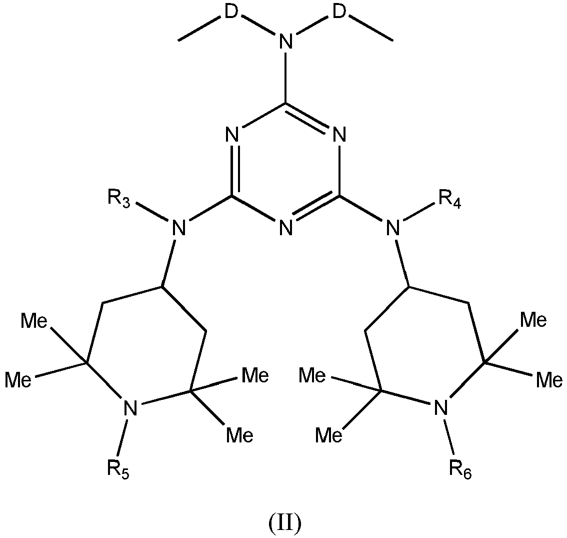 wherein:
D is an optionally unsaturated C₁-C₆ alkylene or isoalkylene group;
R₃, R₄, which can be the same or different, are hydrogen or C₁-C₈ alkyl;
R₅, R₆, which can be the same or different, are hydrogen or straight or branched C₁-C₄ alkyl or an -OG group wherein
G is hydrogen or straight or branched, saturated or unsaturated C₁-C₁₀ alkyl.

2. Compounds according to claim 1
wherein:
n is an integer ranging from 2 to 100;
R₁ and R₂ are hydrogen or C₁-C₄ alkyl;
A is a C₁-C₂₀ alkylene or C₃-C₁₀ cycloalkylene group optionally containing one or more unsaturations;
and B is a group of formula (II): 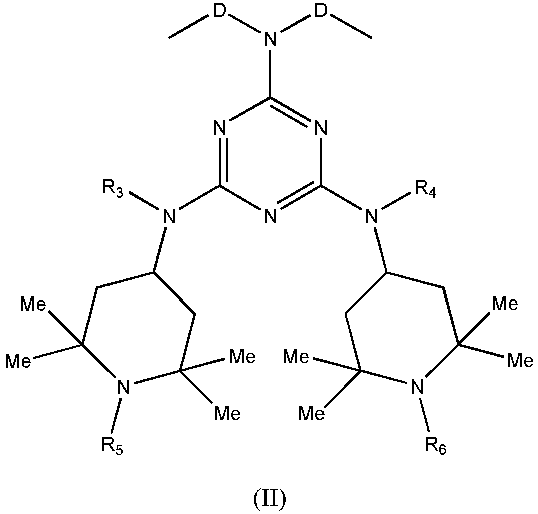 wherein:
D is a C₁-C₆ alkylene or isoalkylene group;
R₃, R₄, which can be the same or different, are hydrogen or C₁-C₈ alkyl;
R₅, R₆, which can be the same or different, are hydrogen or C₁-C₂ alkyl.

3. Compounds according to claim 1 or 2 wherein:
n ranges from 3 to 20,
A is a straight C₁-C₂₀ alkylene group,
D is a -CH₂-CH₂-, -CH₂-CH₂-CH₂-, -CH(CH₃)-CH₂- or -CH₂-CH(CH₃)- group,
R₁ and R₂ are methyl or ethyl,
R₃ and R₄ are hydrogen or n-butyl,
R₅ and R₆ are hydrogen or methyl.

4. Compounds according to any one of claims 1 to 3 wherein:
n ranges from 3 to 20,
A is a -(CH₂)₈- group
D is a -CH₂-CH₂-, -CH(CH₃)-CH₂- or -CH₂-CH(CH₃)- group,
R₁ and R₂ are methyl,
R₃ and R₄ are n-butyl,
R₅ and R₆ are hydrogen or methyl.

5. Mixtures of compounds according to claims 1 to 4.

6. Mixtures of compounds according to claims 1 to 5, and at least one of the compounds of formulas **(P), (Q), (R), (S), (T), (U)** 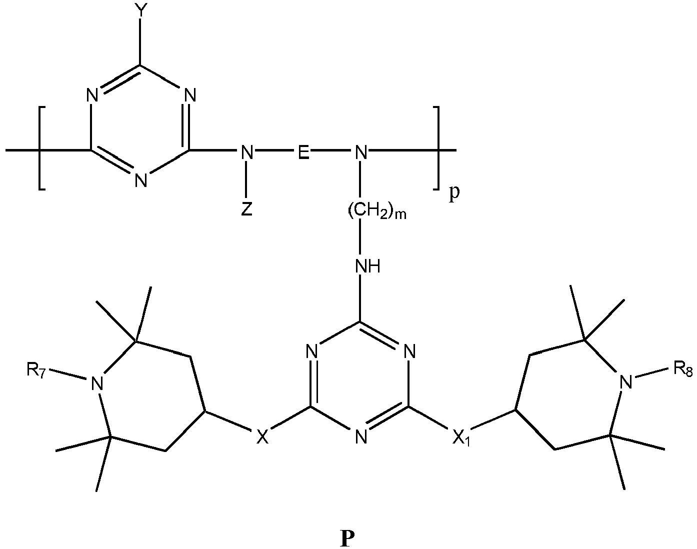
wherein p ranges from 3 to 20;
m ranges from 2 to 12;
R₇ and R₈, which can be the same or different, are hydrogen, a straight or branched C₁-C₁₂ alkyl group, a C₃-C₈ alkenyl group or a C₇-C₁₉ aralkyl group;
X and X₁, which can be the same or different, are oxygen or a group of formula (VIII) 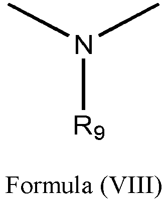 wherein R₉ is hydrogen, a straight or branched C₁-C₁₂ alkyl group, a C₅-C₁₂ cycloalkyl group or a C₇-C₁₂ aralkyl group;
E is a -(CH₂)ₐ- group wherein a ranges from 2 to 12, with the proviso that a is different from m;
Z is a C₁-C₁₈ alkyl group or a group of formula (IX) 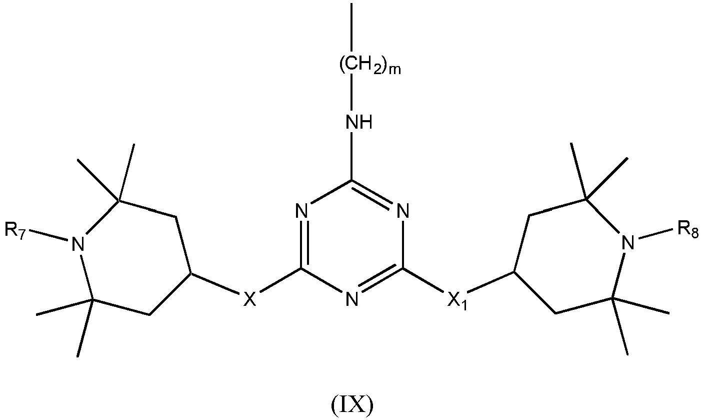 wherein m, X, X₁, R₇ and R₈ are as defined above,
or a group of formula (X) 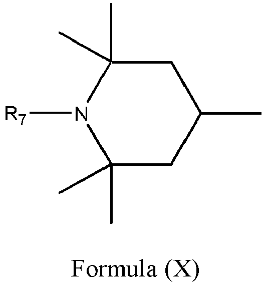 wherein R₇ is as defined above;
Y is an O-R₁₁ or S-R₁₁ group or a group of formula (XI) 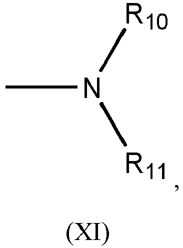 wherein R₁₀ and R₁₁, which can be the same or different, can be hydrogen, a straight or branched C₁-C₁₈ alkyl group, a C₅-C₁₂ cycloalkyl group, a C₇-C₁₂ aralkyl group or a C₆-C₁₂ aryl group or can form, together with the nitrogen atom they are linked to , a morpholino or C₅-C₇ heterocycle group;
and the piperidino group (XII) 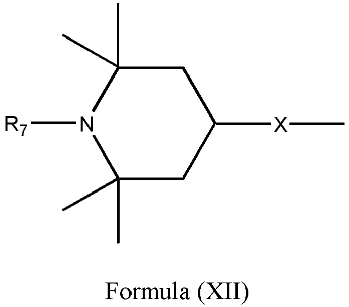 wherein R₇ and X are as defined above; 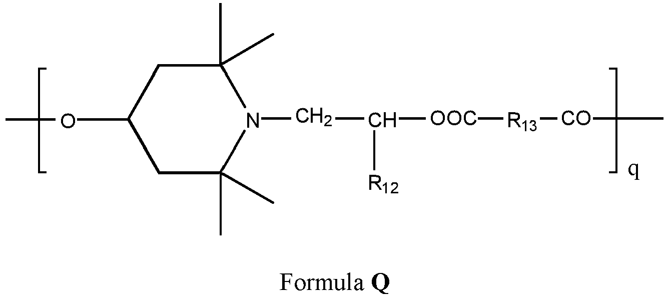 wherein
R₁₂ is selected from the group formed by hydrogen and methyl;
R₁₃ is a direct bond or a C₁-C₁₀ alkylene group;
q is an integer ranging from 2 to 50; 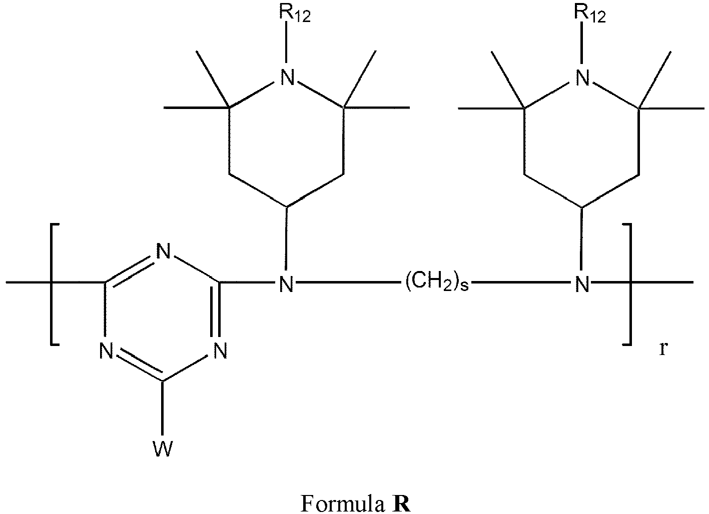
wherein:
r is an integer ranging from 2 to 50;
s is an integer ranging from 2 to 10;
R₁₂ is as defined above for the compounds of formula **Q;**
W is selected from the following groups of formulas (XIII), (XIV) and (XV): 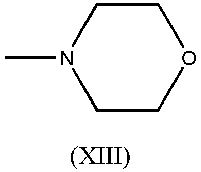 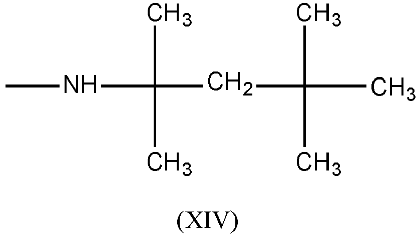 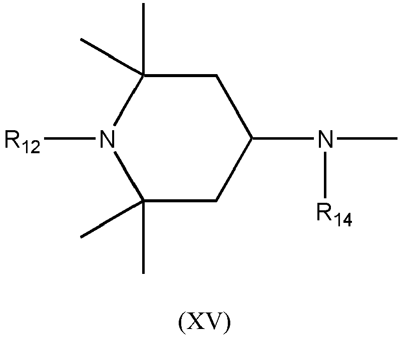 wherein:
R₁₄ is selected from the group formed by straight or branched C₁-C₄ alkyl groups;
R₁₂ is as defined above for the compounds of formula **R;** 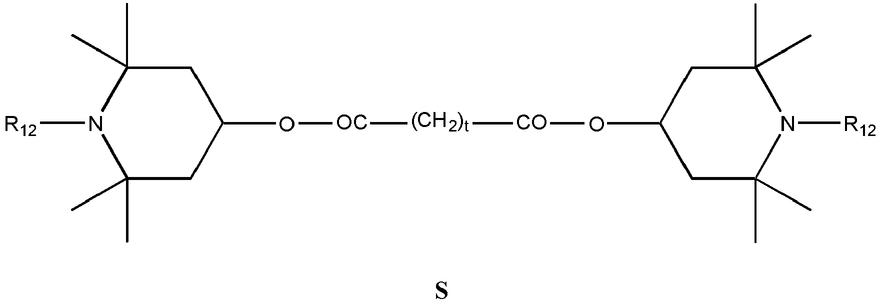
wherein:
t is an integer ranging from 2 to 10;
R₁₂ is as defined above for the compounds of formula R; 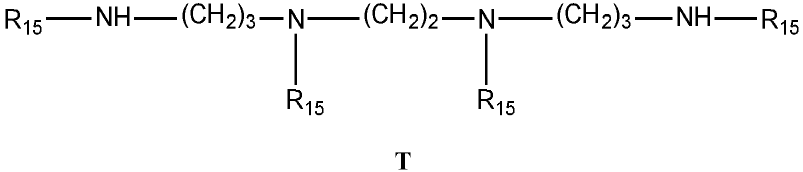
wherein:
R₁₅ is the group of formula (XVI) 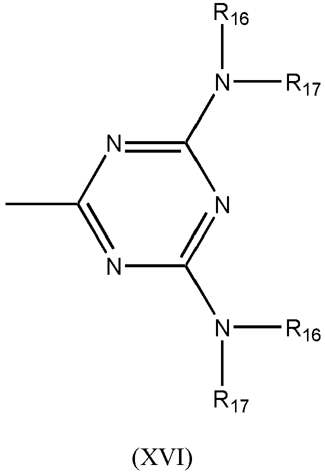 wherein R₁₆ and R₁₇ are independently selected from the group formed by hydrogen, straight or branched C₁-C₄ alkyl groups and the group of formula (XVII) 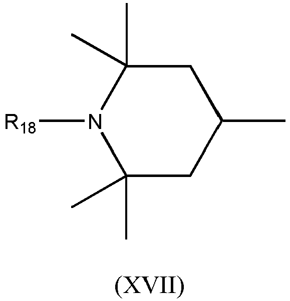 wherein R₁₈ is hydrogen, a straight or branched C₁-C₄ alkyl group or OR₁₉ groups wherein R₁₉ is hydrogen or a straight or branched C₁-C₈ alkyl group; 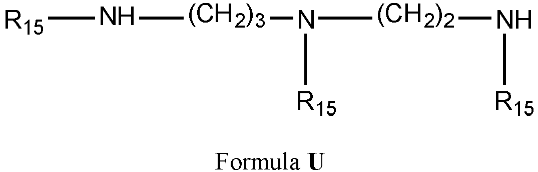 wherein R₁₅ has the meanings defined above for the compounds of formula **T.**

7. Mixtures according to claim 6 comprising 10% to 90% by weight of the compounds of formula (I).

8. Mixtures according to claims 6-7 further comprising antioxidants; UV absorbers; nickel stabilisers; plasticisers, lubricants, antistatic agents, flame retardants, corrosion inhibitors, metal deactivators or mineral fillers.

9. Use of the compounds according to claims 1 to 4 as stabilising agents for polymers.

10. Use of the mixtures of claims 5-6 as stabilising agents for polymers.

11. Use according to claim 9 or 10 for the stabilisation of polyethylene, polypropylene, polystyrene, polybutadiene, polyisoprene and copolymers thereof, polyvinyl chloride, polyvinylidene chloride and copolymers thereof, polyvinyl acetate and copolymers thereof with ethylene; polyesters; polyamides and polyurethanes.

## Patentansprüche

1. Verbindungen der allgemeinen Formel (I): 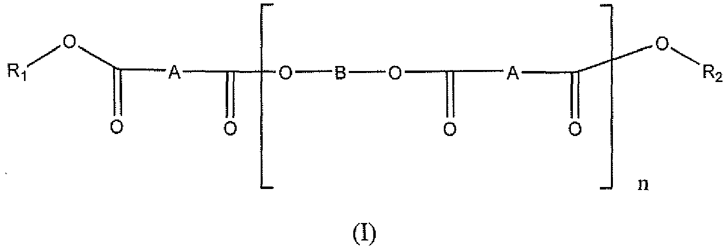 wobei gilt:
n ist eine ganze Zahl im Bereich von 2 bis 100;
R₁ und R₂ sind Wasserstoff oder geradkettige oder verzweigte, gesättigte oder ungesättigte C₁-C₁₈-Alkylgruppen oder aromatische Gruppen;
A ist eine C₁-C₂₀-Alkylen- oder C₃-C₁₀-Cycloalkylengruppe, optional enthaltend eine oder mehr Unsättigungen, oder eine aromatische Gruppe;
und B ist eine Gruppe der Formel (II): 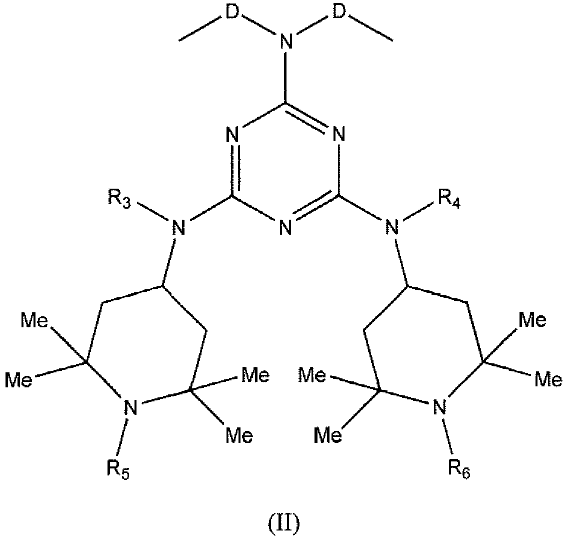 wobei gilt:
D ist eine optional ungesättigte C₁-C₆-Alkylen- oder Isoalkylengruppe;
R₃, R₄, die gleich oder verschieden sein können, sind Wasserstoff oder C₁-C₈-Alkyl;
R₅, R₆, die gleich oder verschieden sein können, sind Wasserstoff oder eine geradkettige oder verzweigte C₁-C₄-Alkyl- oder eine -OG-Gruppe, wobei gilt:
G ist Wasserstoff oder geradkettiges oder verzweigtes, gesättigtes oder ungesättigtes C₁-C₁₀-Alkyl.

2. Verbindungen nach Anspruch 1,
wobei gilt:
n ist eine ganze Zahl im Bereich von 2 bis 100;
R₁ und R₂ sind Wasserstoff oder C₁-C₄-Alkyl;
A ist eine C₁-C₂₀-Alkylen- oder C₃-C₁₀-Cycloalkylengruppe, optional enthaltend eine oder mehr Unsättigungen;
und B ist eine Gruppe der Formel (II): 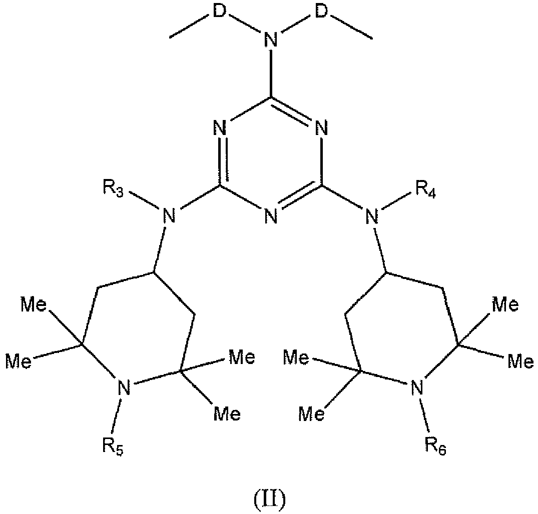 wobei gilt:
D ist eine C₁-C₆-Alkylen- oder Isoalkylengruppe;
R₃, R₄, die gleich oder verschieden sein können, sind Wasserstoff oder C₁-C₈-Alkyl;
R₅, R₆, die gleich oder verschieden sein können, sind Wasserstoff oder C₁-C₂-Alkyl.

3. Verbindungen nach Anspruch 1 oder 2, wobei gilt:
n liegt im Bereich von 3 bis 20,
A ist eine geradkettige C₁-C₂₀-Alkylengruppe,
D ist eine -CH₂-CH₂-, -CH₂-CH₂-CH₂-, -CH(CH₃)-CH₂- oder -CH₂-CH(CH₃)-Gruppe,
R₁ und R₂ sind Methyl oder Ethyl,
R₃ und R₄ sind Wasserstoff oder n-Butyl,
R₅ und R₆ sind Wasserstoff oder Methyl.

4. Verbindungen nach einem der Ansprüche 1 bis 3
wobei gilt:
n liegt im Bereich von 3 bis 20,
A ist eine -(CH₂)₈-Gruppe
D ist eine -CH₂-CH₂-, -CH(CH₃)-CH₂- oder -CH₂-CH(CH₃)-Gruppe,
R₁ und R₂ sind Methyl,
R₃ und R₄ sind n-Butyl,
R₅ und R₆ sind Wasserstoff oder Methyl.

5. Gemische aus Verbindungen nach den Ansprüchen 1 bis 4.

6. Gemische aus Verbindungen nach den Ansprüchen 1 bis 5, und mindestens einer der Verbindungen der Formeln **(P), (Q), (R), (S), (T), (U)** 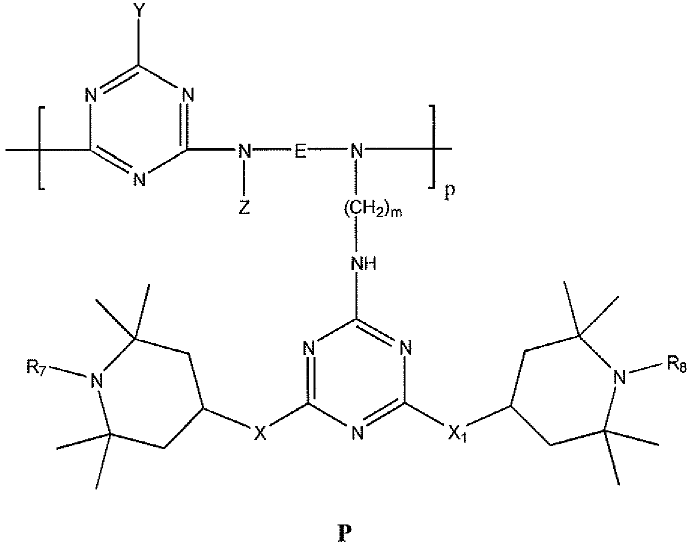
wobei gilt: p liegt im Bereich von 3 bis 20;
m liegt im Bereich von 2 bis 12;
R₇ und R₈, die gleich oder verschieden sein können, sind Wasserstoff, eine geradkettige oder verzweigte C₁-C₁₂-Alkylgruppe, eine C₃-C₈-Alkenylgruppe oder eine C₇-C₁₉-Aralkylgruppe;
X und X₁, die gleich oder verschieden sein können, sind Sauerstoff oder eine Gruppe der Formel (VIII) 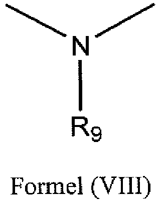 wobei gilt: R₉ ist Wasserstoff, eine geradkettige oder verzweigte C₁-C₁₂-Alkylgruppe, eine C₅-C₁₂-Cycloalkylgruppe oder eine C₇-C₁₂-Aralkylgruppe;
E ist eine -(CH₂)ₐ-Gruppe, wobei a im Bereich von 2 bis 12 liegt, mit der Maßgabe, dass a verschieden von m ist;
**Z** ist eine C₁-C₁₈-Alkylgruppe oder eine Gruppe der Formel (IX) 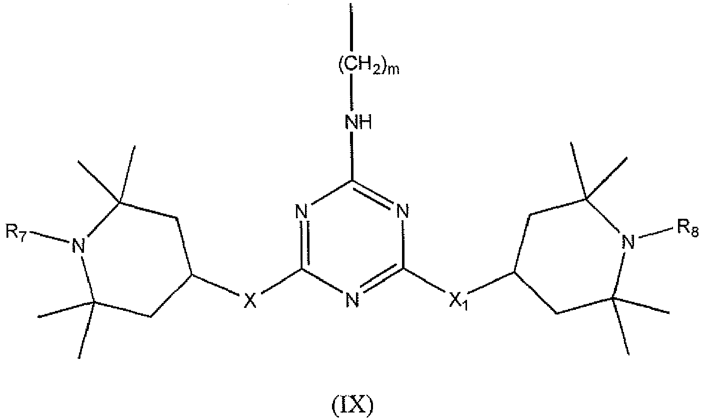 wobei gilt: m, X, X₁, R₇ und R₈ sind wie oben definiert,
oder eine Gruppe der Formel (X) 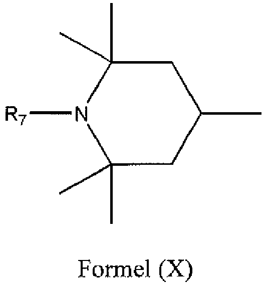 wobei gilt: R₇ ist wie oben definiert;
Y ist eine O-R₁₁- oder S-R₁₁-Gruppe oder eine Gruppe der Formel (XI) 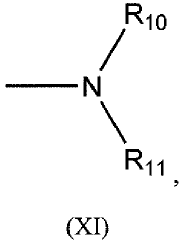 wobei gilt: R₁₀ und R₁₁, die gleich oder verschieden sein können, können sein: Wasserstoff, eine geradkettige oder verzweigte C₁-C₁₈-Alkylgruppe, eine C₅-C₁₂-Cycloalkylgruppe, eine C₇-C₁₂-Aralkylgruppe oder eine C₆-C₁₂-Arylgruppe, oder können, zusammen mit dem Stickstoffatom, mit dem sie verbunden sind, eine Morpholino- oder C₅-C₇-heterocyclische Gruppe bilden;
und die Piperidinogruppe (XII) 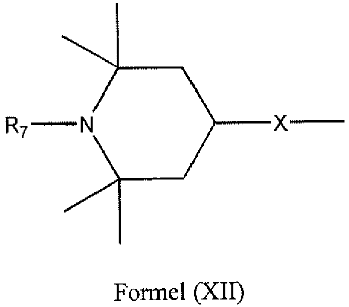 wobei gilt: R₇ und X sind wie oben definiert; 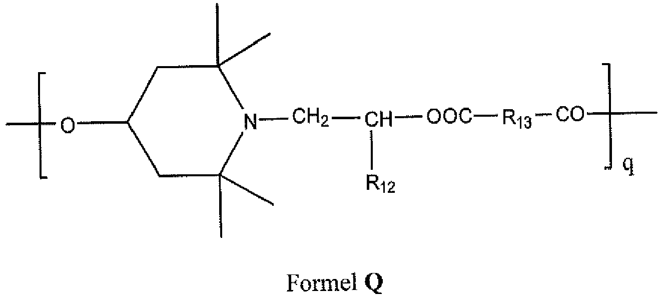 wobei gilt:
R₁₂ ist ausgewählt aus der Gruppe, bestehend aus Wasserstoff und Methyl;
R₁₃ ist eine direkte Bindung oder eine C₁-C₁₀-Alkylengruppe;
q ist eine ganze Zahl im Bereich von 2 bis 50; 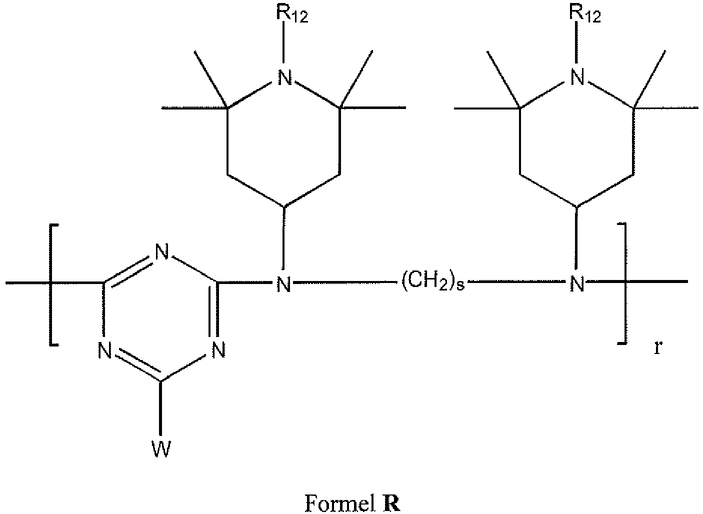
wobei gilt:
r ist eine ganze Zahl im Bereich von 2 bis 50;
s ist eine ganze Zahl im Bereich von 2 bis 10;
R₁₂ ist wie oben für die Verbindungen der Formel **Q** definiert;
W ist ausgewählt aus den folgenden Gruppen von Formeln (XIII), (XIV) und (XV): 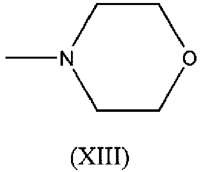 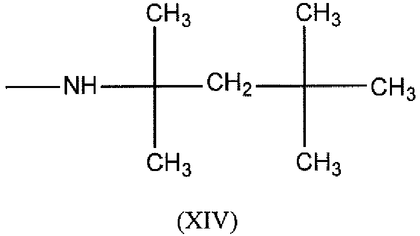 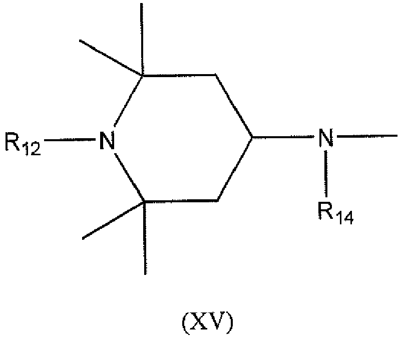 wobei gilt:
R₁₄ ist ausgewählt aus der Gruppe, gebildet durch geradkettige oder verzweigte C₁-C₄-Alkylgruppen;
R₁₂ ist wie oben für die Verbindungen der Formel **R** definiert; 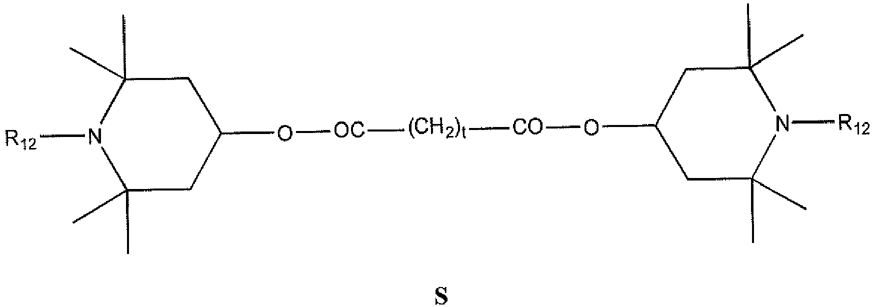
wobei gilt:
t ist eine ganze Zahl im Bereich von 2 bis 10;
R₁₂ ist wie oben für die Verbindungen der Formel **R** definiert; 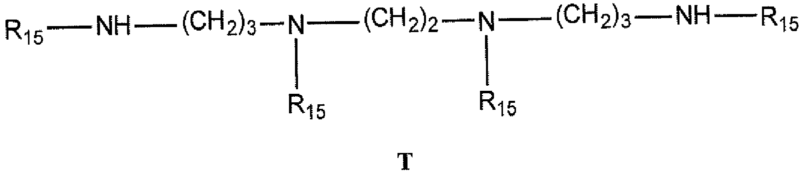
wobei gilt:
R₁₅ ist die Gruppe der Formel (XVI) 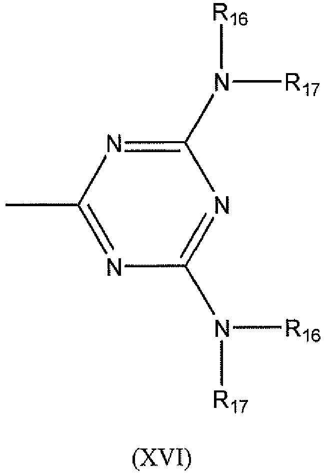 wobei gilt: R₁₆ und R₁₇ sind unabhängig ausgewählt aus der Gruppe, gebildet durch Wasserstoff, geradkettige oder verzweigte C₁-C₄-Alkylgruppen und die Gruppe der Formel (XVII) 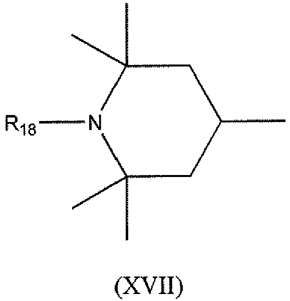 wobei gilt: R₁₈ ist Wasserstoff, eine geradkettige oder verzweigte C₁-C₄-Alkylgruppe oder OR₁₉-Gruppen, wobei gilt: R₁₉ ist Wasserstoff oder eine geradkettige oder verzweigte C₁-C₈-Alkylgruppe; 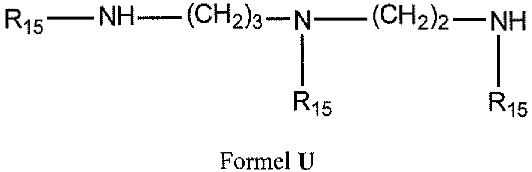 wobei gilt: R₁₅ hat die oben für die Verbindungen der Formel **T** definierten Bedeutungen.

7. Gemische nach Anspruch 6, umfassend 10 Gew.-% bis 90 Gew.-% der Verbindungen der Formel (I).

8. Gemische nach den Ansprüchen 6-7, des Weiteren umfassend Antioxidantien; UV-Absorber; Nickelstabilisatoren; Weichmacher, Schmiermittel, antistatische Mittel, Flammverzögerungsmittel, Korrosionsinhibitoren, Metalldeaktivatoren oder mineralische Füllstoffe.

9. Verwendung der Verbindungen nach den Ansprüchen 1 bis 4 als Stabilisierungsmittel für Polymere.

10. Verwendung der Gemische der Ansprüche 5-6 als Stabilisierungsmittel für Polymere.

11. Verwendung nach Anspruch 9 oder 10 zur Stabilisierung von Polyethylen, Polypropylen, Polystyren, Polybutadien, Polyisopren und Copolymeren davon, Polyvinylchlorid, Polyvinylidenchlorid und Copolymeren davon, Polyvinylacetat und Copolymeren davon mit Ethylen; Polyestern; Polyamiden und Polyurethanen.

## Revendications

1. Composés répondant à la formule générale (I) : 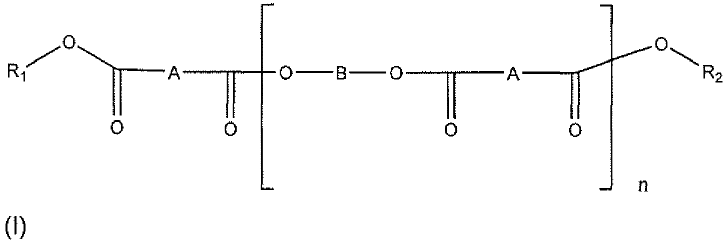 dans laquelle :
n représente un nombre entier se trouvant dans la plage allant de 2 à 100 ;
R₁ et R₂ représentent un hydrogène ou des groupes alkyle en C₁ à C₁₈ linéaires ou ramifiés, saturés ou insaturés ou des groupes aromatiques ;
A représente un groupe alkylène en C₁ à C₂₀ ou cycloalkylène en C₃ à C₁₀ contenant éventuellement une ou plusieurs insaturation(s), ou un groupe aromatique ;
et B représente un groupe répondant à la formule (II) : 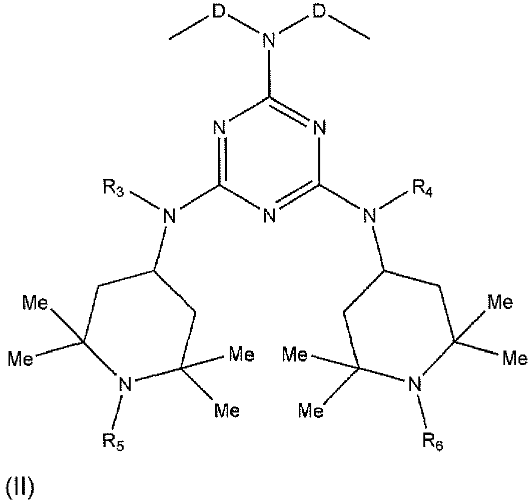 dans laquelle :
D représente un groupe isoalkylène ou alkylène en C₁ à C₆ éventuellement insaturé ;
R₃, R₄, qui peuvent être identiques ou différents, représentent un hydrogène ou un alkyle en C₁ à C₈ ;
R₅, R₆, qui peuvent être identiques ou différents, représentent un hydrogène ou un alkyle en C₁ à C₄ linéaire ou ramifié ou un groupe -OG dans lequel
G représente un hydrogène ou un alkyle en C₁ à C₁₀ linéaire ou ramifié, saturé ou insaturé.

2. Composés selon la revendication 1
dans lesquels :
n représente un nombre entier se trouvant dans la plage allant de 2 à 100 ;
R₁ et R₂ représentent un hydrogène ou un alkyle en C₁ à C₄ ;
A représente un groupe alkylène en C₁ à C₂₀ ou cycloalkylène en C₃ à C₁₀ contenant éventuellement une ou plusieurs insaturation(s) ;
et B représente un groupe répondant à la formule (II) : 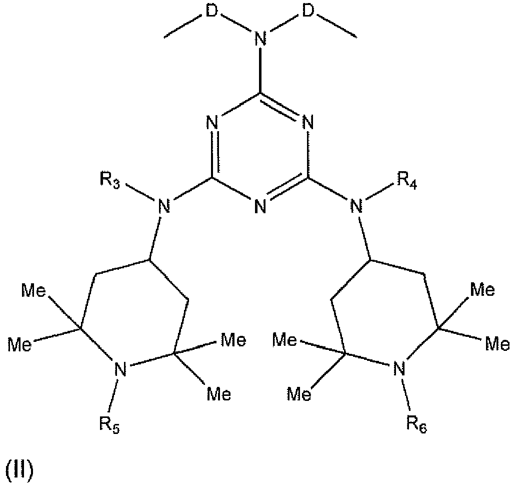 dans laquelle :
D représente un groupe isoalkylène ou alkylène en C₁ à C₆ ;
R₃, R₄, qui peuvent être identiques ou différents, représentent un hydrogène ou un alkyle en C₁ à C₈ ;
R₅, R₆, qui peuvent être identiques ou différents, représentent un hydrogène ou un alkyle en C₁ ou C₂.

3. Composés selon la revendication 1 ou 2, dans lesquels :
n se trouve dans la plage allant de 3 à 20,
A représente un groupe alkylène en C₁ à C₂₀ linéaire,
D représente un groupe -CH₂-CH₂-, -CH₂-CH₂-CH₂-, -CH(CH₃)-CH₂- ou-CH₂-CH(CH₃)-,
R₁ et R₂ représentent un méthyle ou un éthyle,
R₃ et R₄ représentent un hydrogène ou un n-butyle,
R₅ et R₆ représentent un hydrogène ou un méthyle.

4. Composés selon l'une quelconque des revendications 1 à 3, dans lesquels :
n se trouve dans la plage allant de 3 à 20,
A représente un groupe -(CH₂)₈-
D représente un groupe -CH₂-CH₂-, -CH(CH₃)-CH₂- ou -CH₂-CH(CH₃)-,
R₁ et R₂ représentent un méthyle,
R₃ et R₄ représentent un n-butyle,
R₅ et R₆ représentent un hydrogène ou un méthyle.

5. Mélanges de composés selon les revendications 1 à 4.

6. Mélanges de composés selon les revendications 1 à 5, et d'au moins l'un des composés répondant aux formules (P), (Q), (R), (S), (T), (U) 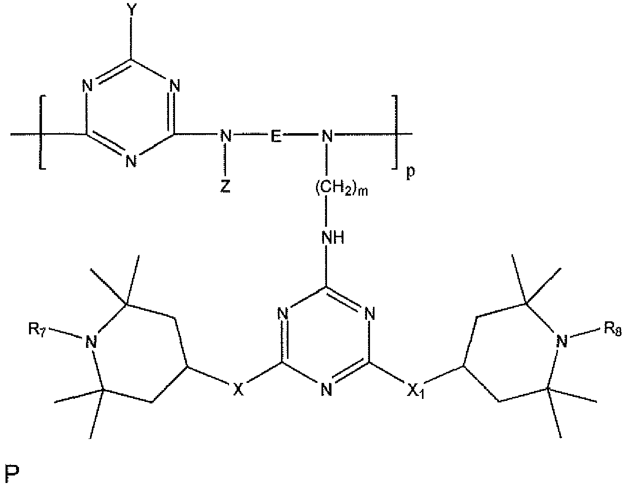
dans laquelle p se trouve dans la plage allant de 3 à 20 ;
m se trouve dans la plage allant de 2 à 12 ;
R₇ et R₈, qui peuvent être identiques ou différents, représentent un hydrogène, un groupe alkyle en C₁ à C₁₂ linéaire ou ramifié, un groupe alcényle en C₃ à C₈ ou un groupe aralkyle en C₇ à C₁₉ ;
X et X₁, qui peuvent être identiques ou différents, représentent un oxygène ou un groupe répondant à la formule (VIII) 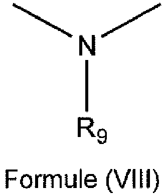 dans laquelle R₉ représente un hydrogène, un groupe alkyle en C₁ à C₁₂ linéaire ou ramifié, un groupe cycloalkyle en C₅ à C₁₂ ou un groupe aralkyle en C₇ à C₁₂ ;
E représente un groupe -(CH₂)ₐ- où a se trouve dans la plage allant de 2 à 12, à condition que a soit différent de m ;
Z représente un groupe alkyle en C₁ à C₁₈ ou un groupe répondant à la formule (IX) 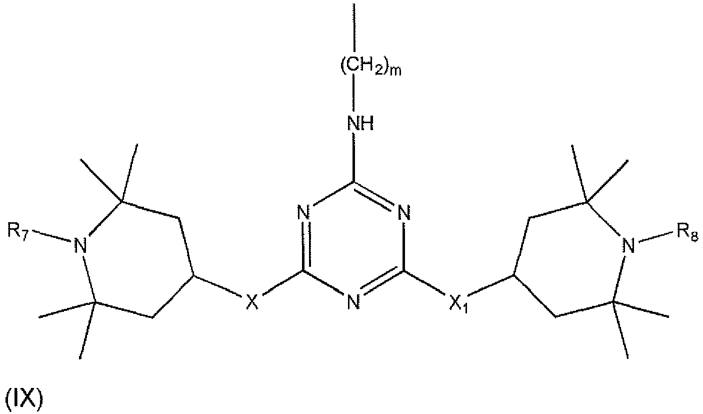 dans laquelle m, X, X₁, R₇ et R₈ sont tels que définis ci-dessus,
ou un groupe répondant à la formule (X) 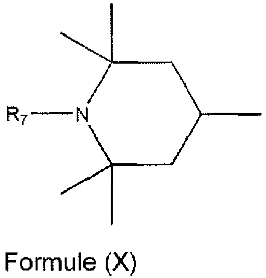 dans laquelle R₇ est tel que défini ci-dessus ;
Y représente un groupe O-R₁₁ ou S-R₁₁ ou un groupe répondant à la formule (XI) 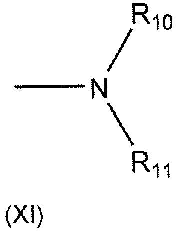 dans laquelle R₁₀ et R₁₁, qui peuvent être identiques ou différents, peuvent être un hydrogène, un groupe alkyle en C₁ à C₁₈ linéaire ou ramifié, un groupe cycloalkyle en C₅ à C₁₂, un groupe aralkyle en C₇ à C₁₂ ou un groupe aryle en C₆ à C₁₂, ou peuvent former, conjointement avec l'atome d'azote auquel ils sont liés, un groupe morpholino ou hétérocycle en C₅ à C₇ ;
et le groupe pipéridino (XII) 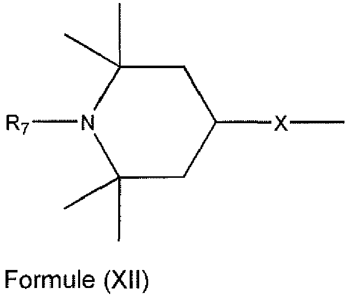 dans laquelle R₇ et X sont tels que définis ci-dessus ; 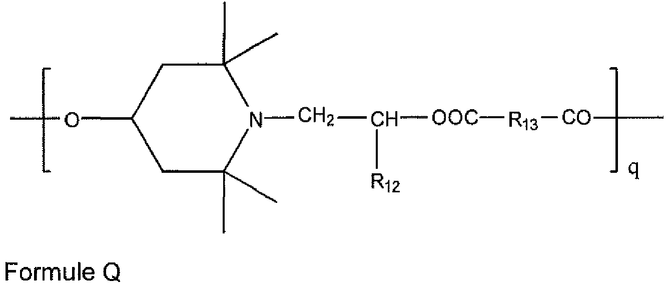 dans laquelle
R₁₂ est choisi dans le groupe formé par un hydrogène et un méthyle ;
R₁₃ représente une liaison directe ou un groupe alkylène en C₁ à C₁₀ ;
q représente un nombre entier se trouvant dans la plage allant de 2 à 50 ; 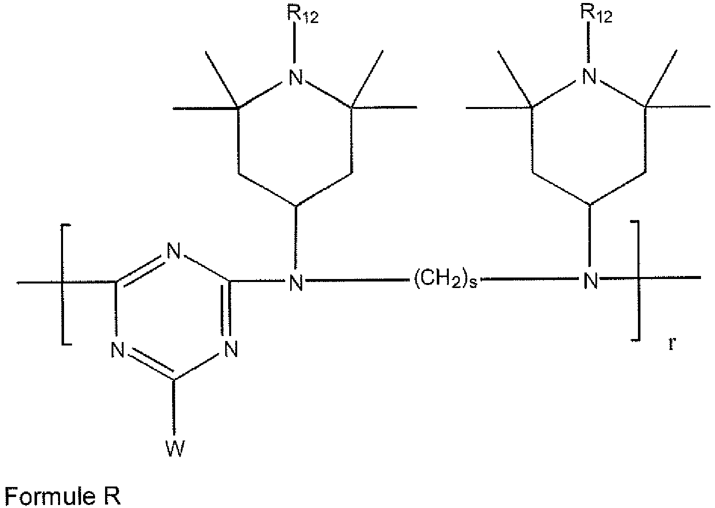
dans laquelle :
r représente un nombre entier se trouvant dans la plage allant de 2 à 50 ;
s représente un nombre entier se trouvant dans la plage allant de 2 à 10 ;
R₁₂ est tel que défini ci-dessus pour les composés répondant à la formule Q ;
W est choisi parmi les groupes suivants répondant aux formules (XIII), (XIV) et (XV) : 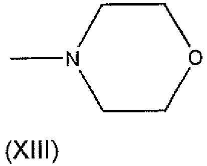 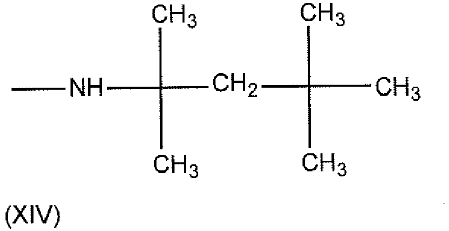 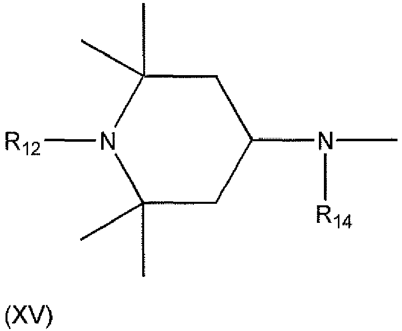
dans lesquelles :
R₁₄ est choisi dans le groupe formé par des groupes alkyle en C₁ à C₄ linéaires ou ramifiés ;
R₁₂ est tel que défini ci-dessus pour les composés répondant à la formule R ; 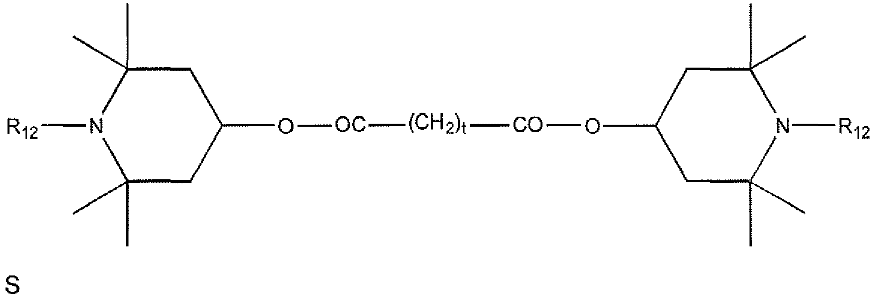 dans laquelle :
t représente un nombre entier se trouvant dans la plage allant de 2 à 10 ;
R₁₂ est tel que défini ci-dessus pour les composés répondant à la formule R ; 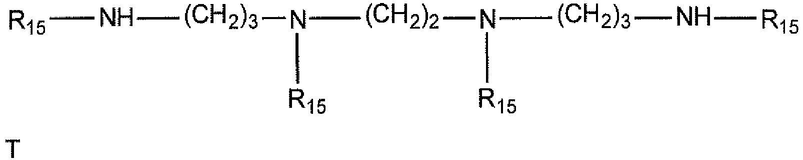
dans laquelle :
R₁₅ représente le groupe répondant à la formule (XVI) 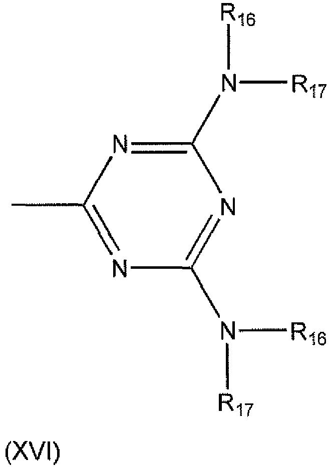 dans laquelle R₁₆ et R₁₇ sont choisis indépendamment dans le groupe formé par un hydrogène, des groupes alkyle en C₁ à C₄ linéaires ou ramifiés et le groupe répondant à la formule (XVII) 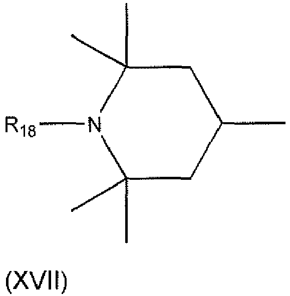 dans laquelle R₁₈ représente un hydrogène, un groupe alkyle en C₁ à C₄ linéaire ou ramifié ou des groupes OR₁₉ dans lesquels R₁₉ représente un hydrogène ou un groupe alkyle en C₁ à C₈ linéaire ou ramifié ; 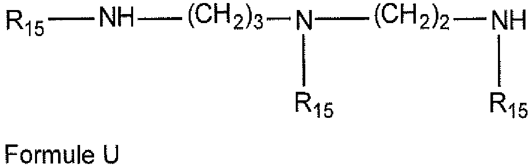 dans laquelle R₁₅ a les significations définies ci-dessus pour les composés répondant à la formule T.

7. Mélanges selon la revendication 6, comprenant 10% à 90% en poids des composés répondant à la formule (I).

8. Mélanges selon les revendications 6 et 7, comprenant en outre des antioxydants ; des absorbeurs UV ; des stabilisants à base de nickel ; des plastifiants, des lubrifiants, des agents antistatiques, des retardateurs de flamme, des inhibiteurs de corrosion, des désactivateurs de métaux ou des charges minérales.

9. Utilisation des composés selon les revendications 1 à 4 en tant qu'agents stabilisants pour polymères.

10. Utilisation des mélanges des revendications 5 et 6 en tant qu'agents stabilisants pour polymères.

11. Utilisation selon la revendication 9 ou 10 pour la stabilisation de polyéthylène, de polypropylène, de polystyrène, de polybutadiène, de polyisoprène et de leurs copolymères, de chlorure de polyvinyle, de chlorure de polyvinylidène et de leurs copolymères, de l'acétate de polyvinyle et de ses copolymères avec l'éthylène ; de polyesters ; de polyamides et de polyuréthannes.
